# EUROPEAN PATENT APPLICATION

(11) **EP 1 219 306 A1**
(43) Date of publication of application: **03.07.2002**
(21) Application number: 00403719.8
(22) Date of filing: 29.12.2000
(51) Int. Cl.: A61K 47/48

(54) **Compositions comprising cyclodextrins and NO- releasing drugs**

(71) Applicant: NICOX S.A., 06560 Valbonne, Sophia Antipolis (FR)
(72) Inventor: Naggi, Annamaria, 20025 Legnano (MI) (IT); Torri, Gian Giacomo, 20133 Milano (IT); Trespidi, Laura, 26026 Pizzighettone (CR) (IT)
(74) Representative: Cleva, Maria Giovanna

(57) **Abstract**

The present invention relates to composition comprising cyclodextrins and a NO-releasing drug of formula

A-X-L-NOₙ

wherein
**A** is the radical deriving from a drug;
**X** is a divalent radical connecting A with the NO-releasing group L-NOₙ;
**L** is selected from the group consisting of: O and S;
**n** is 1 or 2.

## Description

### Field of the Invention

The present invention relates to compositions comprising a NO-releasing derivative of a pharmaceutically active compound.

### Background of the Invention

In the last decade there has been a growing interest towards the preparation and the properties of compounds comprising a radical derived from a compound having pharmaceutical activity and a NO releasing group.
EP 670 82, EP 759 899 and EP 722 434 disclose nitric esters of non-steroidal antiinflammatory drugs (NSAIDs). These compounds present an improved activity and reduced side effects when compared to the drug without NO-releasing group.
WO 98/15568 discloses nitrate esters of corticoids. Also in this case a reduced toxicity is observed when the nitrate group is present.
Compounds comprising a radical derived from an antithrombotic drug and a NO-releasing group are described in WO 98/21193. The comparative data show that the introduction of the NO-releasing group causes an increase of activity of the drug.
WO 00/61537 discloses the preparation of drugs comprising a NO releasing group linked to, inter alia, anti-inflammatory, analgesic, bronchodilators, ACE-inhibitors, β-blockers, antineoplastic compounds. The use of a linking group presenting specific antioxidant properties allows the use of these drugs to patients affected by oxidative stress and/or endothelial dysfunction.
Thus, it is possible to say that the introduction of NO releasing groups has proven to be advantageous in many classes of drugs. However, the introduction of a NO releasing group often leads to a relevant drawback, i.e. a significant reduction in water solubility, that might lead to a slower adsorption rate of the drug in the human body. It is therefore desirable to find methods to improve the bioavailability of compounds comprising a radical derived from a compound having pharmaceutical activity and a NO-releasing group.
The use of cyclodextrin complexes in combination with NO releasing compounds is known from WO 95/29172. In that case, however, there was no radical derived from a compound having pharmaceutical activity in the molecule complexed with Cyclodextrin and, furthermore, the problem was to render the molecule stable to degradation. Thus, both the type of compound and the technical problem solved by the patent application are quite different from the present case.

### Summary of the invention

The present invention relates to compositions for pharmaceutical use comprising a cyclodextrin and a compound comprising a radical derived from a compound having pharmaceutical activity and a NO releasing group.

### Detailed description of the invention

The invention relates to compositions comprising cyclodextrins and a NO-releasing drug of formula

A-X-L-NOₙ

wherein
**A** is the radical deriving from a drug;
**X** is a divalent radical connecting A with the NO-releasing group;
**L** is selected from the group consisting of: O and S; preferably it is O;
**n** is 1 or 2, preferably it is 2.
The syntheses of these compounds is described in the following patents, which are herewith incorporated by reference: US 5,861,426, WO 98/15568, US 5,621,000, WO 00/61537, WO 00/61541, WO 00/61604, US 5,703,073, US 6,043,233, US 6,057,347.
Cyclodextrins are cyclic oligosaccharides constituted by the union of from 6 to 12 glucose units through α(1,4) bonds. The word CD, used to indicate them, is usually preceded by a Greek letter that indicates the amount of glucose units (α corresponds to 6, β corresponds to 7, and so on).
A characteristic parameter of CDs is the diameter of the cavity wherein the compound is complexed.
For many purposes α-CD have a too small cavity (5 Å) to complex molecules of a medium size.
This is why for many applications β-CD is preferred (diameter: 6 Å). The drawback of β-CD is its low solubility in water (18.5 g/l). To overcome the problem, probably caused by inter- and intramolecular hydrogen bonds between the hydroxyl groups, β CD derivatives have been prepared which present a considerably higher water solubility. In fact, it is known that the hydroxyl groups in the glucose units of CDs can be selectively reacted to prepare ethers, esters, ionic ethers (see for example the review "Physicochemical Characteristics and Pharmaceutical uses of Cyclodextrin Derivatives" D. Duchene et al., Pharmacueutical Technology International, June 1990).
The cyclodextrins to be used in combination with the compounds of formula A-X-L-NOₙ are not particularly limited. Preferred examples of cyclodextrins useful in the present invention are: α-CD, dimethyl α-CD, trimethyl α-CD, β-CD, dimethyl β-CD, trimethyl β-CD, 2-hydroxypropyl β-CD, 3-hydroxypropyl β-CD, 2,3-dihydroxypropyl β-CD, γ-CD, dimethyl γ-CD, trimethyl γ-CD and polymeric CD.
In each particular case, it is possible to determine, with a few trials, which one is the most suitable cyclodextrin to be used in combination with a specific drug.
The molar ratio between the drug and the cyclodextrin can vary in a broad range. Preferably it is comprised between 1:10 and 10:1, more preferably between 3:1 and 1:3.
The composition according to the invention can be prepared in different ways. For example, it is possible to mix together the cyclodextrin and the NO-releasing drug in water. Due to the low solubility of most drugs, the drug is partly or fully dissolved when complexed with the CD. The solution is then dried and the solid recovered. It is also possible to use a cosolvent (e.g. ethanol) which is miscible with water and that solubilizes the drug. In another embodiment it is also possible to isolate the pure complex by using a two phase system: a lipophilic solvent wherein the drug is soluble, and water. The CD dissolves in the water phase, the drug in the lipophilic pahse. The complex CD-drug is formed at the interphase. If it is soluble in water, it is recovered from the water phase.
Finally, it is also possible to simply mix the drug and the CD in the solid state by using mixing and/or milling means well known in the art.
In a preferred embodiment, the drug used in the compositions according to the present invention, is selected from the following classes of compounds:
non steroidal antiinflammatory and analgesic drugs, antibacterial (antibiotics), antiviral, steroids, antineoplastic, β-adrenergics (agonists and blockers), antihyperlipoproteinemic, bone resorption inhibitors.

Non limiting examples of non-steroidal anti-inflammatory and analgesic drugs are:
Aspirin, Salicylic acid, Mesalamine, Acetylsalicylsalicylic acid, Paracetamol, Etodolac, Pirazolac, Tolmetin, Bromefenac, Fenbufen, Mofezolac, Diclofenac, Pemedolac, Sulindac, Ketorolac, Indomethacin, Suprofen, Ketoprofen, Tiaprofenic acid, Fenoprofen, Indoprofen, Carprofen, Naproxen, Loxoprofen, Ibuprofen, Pranoprofen, Bermoprofen, CS-670, Zaltoprofen, Tenoxicam, Piroxicam, Meloxicam, Tenidap, Aceclofenac, Acemetacin, 5-amino-acetylsalicylic acid, Alclofenac, Alminoprofen, Amfenac, Bendazac, α-bisabolol, Bromosaligenin, Bucloxic acid, Butibufen, Cinmetacin, Clidanac, Clopirac, Diflunisal, Ditazol, Enfenamic acid, Etofenamate, Felbinac, Fenclozic acid, Fendosal, Fentiazac, Fepradinol, Flufenamic acid, Flunixin, Flunoxaprofen, Flurbiprofen, Glucametacin, Glycol salicilate, Ibuproxam, Isofezolac, Isoxepac, Isoxicam, Lornoxicam, Meclofenamic acid, Mefenamic acid, Metiazinic acid, Niflunic acid, Oxaceprol, Oxaprozin, Oxyphenbutazone, Parsalmide, Perisoxal, Olsalazine, Pirprofen, Protizinic acid, Salacetamide, Salicilamide O-acetic acid, Salsalate, Suxibuzone, Tiaramide, Tinoridine, Tolfenamic acid, Tropesin, Xenbucin, Ximoprofen, Zomepirac, Tomoxiprol.

Non limiting examples of antibacterials (antibiotics) are:
Metronidazolo, Ethambutol, Cycloserina, Cloxyquin, Negamycin, Nitroxoline, Mupirocin, Myxin, Novobiocin, Spectinomycin, Sulbactam, Tigemonam, Tubercidin, Nifurpirinol, Nifurprazine, Glyconiazide, Isoniazide, Opiniazide, Clofazamine, Meclocycline, Minocycline, Sancicline, Tetracicline, Oxytretracycline, Chlortetracycline, Demeclocycline, Methacycline, Doxicycline, Clomocycline, Cinoxacin, Rolitetraciclyne, Pipaciclyne, Guamecycline, Lymecyclinem, Apiciclyne, Nalidixic acid, Cyprofloxacin, Enoxacin, Floroxacin, Pipemidic acid, Difloxacin, Perfloxacin, Enrofloxacin Nadifloxacin, Grepafloxacin, Lomefloxacin, Sparfloxacin, Clinafloxacin, Tosufloxacin, Trovafloxacin, Ofloxacin, Flumequine, Pazufloxacin, Rufloxacin, Norfloxacin, Cefroxadine, Cephradine, Cefaclor, Cefadroxil, Cefprozil Cefatrizine, Cefpiramide, Cephalexin, Cephaloglycin, Loracarbef, Pivcephalexin, Cephamandole, Moxalactam, Cefclidin, Cefepime, Cefuzopran, Ceftibuten, Cefpodoxime Proxetil, Cefotaxime, Cefcapene Pivoxil, Cefodizime, Ceftiofur, Ceftriaxone, Cefditoren, Cefmenoxime, Cefteram, Cefuzonam, Cefdinir, Cefetamet, Cefixime, Cefpirome, Ceftazidine, Cefminox, Cephalosporin, Cefotiam, Ceforanide, Cefazolin, Ceftizoxime, Cefazedone, Cefonicid, Ceftezole, Cephacetrile, Cephapirin, Fenbenicillin, Hetacillin, Quinacillin, Pivampicillin, Aspoxicillin, Mezlocillin, Amoxicillin, Ampicillin, Epicillin, Phenethamate Cyclacillin, Amdinocillin, Penicillin N, Apalcillin, Bacampicillin, Sultamicillin, Talampicillin, Lenampicillin, Benzyl penicillic acid, Carbenecillin, Carindacillin, Clometocillin, Cloxacillin, Dicloxacillin, Floxacillin, Metampicillin, Methicillin, Oxacillin, Penicillin O, Penicillin V, Pheneticillin, Piperacillin, Propicillin, Sulbenicillin, Ticarcillin, Meropenem, Panipenem, Imipenem, Aztreonam, Carumonan, Sulfabenzamide, Sulfacetamide, Sulfachloropyridazine, Sulfacytine, Sulfadiazine, 4'-(Methylsulfamoyl)sulfanilanilide, Sulfadicramide, Sulfadoxine, Sulfamethoxine, Sulfaethidolo, Sulfaguanole, Sulfalene, Sulfamerazine, Sulfameter, Sulfamethazine, Sulfamethizolo, Sulfamethonide, Sulfamethoxazole, Sulfamethoxypyridazine, Sulfamethylthiazole, Sulfametrole, Sulfamoxolo, Sulfanilamide, N⁴-Sulfanilylsulfanilamide, Sulfanilyurea, N-Sulfanil-3,4-xylamide, Sulfaperine, Sulfaphenazole, Sulfaproxyline, Sulfapyrazine, Sulfapyridine, 4-Sulfanilamido salicylic acid, Sulfasomizole, Sulfasymazine, Sulfathiazole, Sulfathiourea, Sulfisomidine, Sulfisoxazole, Acetyl sulfamethoxypyrazine, Sulfaguanidine, Mafenide, Succisulfone, p-Sulfanylbenzylamine, Dapsone, Acediasulfone, Thiazolsulfone, 2-p-Sulfanilylanilino-ethanol, Benzylsulfamide, p-Aminosalicylic acid, p-Aminosalicylic acid hydrazide, Phenyl aminosalicylate, 4-4'-sulfinyldianiline, Clindamycin, Lincomycin, Josamycin, Midecamycins, Rokitamycin, Spiramycins, Mikamycin B, Rosaramycin, Azithromycin, Clarithromycin, Erytromycin, Dirithromycin, Amikacin, Arbekacin, Dibekacin, Tobramycin, Dihydrostreptomycin, Streptomycin, Deoxydihydrostreptomycin, Trospectomycin, Spectinomycin, Micronomicin, Netilmicin, Apramycin, Sisomicin, Neomycin, Paromomycin, Ribostamycin, Rifampin, Rifapentine. Sulfachrysoidine, Sulfamidochrysoidine, Salazosulfadimidine.

Non limiting examples of antiviral drugs are:
Acyclovir, Amantadine, Cidofovir, Cytarabine, Didanosine, Dideoxyadenosine, Edoxuridine, Famciclovir, Floxuridine, Ganciclovir, Idoxuridine, Indanavir, Lamivudine, Kethoxal, MADU, Penciclovir, Ribavirin, Sorivudine, Stavudine, Trifluridine, Valacyclovir, Vidarabine, Xenazoic acid, Zaltacitabine, Zidovudine.

Non limiting examples of steroids are:
Budesonide, Hydrocortisone, Aclomethasone, Algestone, Beclomethasone, Betamethasone, Chlorprednisone, Clobetasol, Clobetasone, Clocortolone, Cloprednol, Cortisone, Corticosterone, Deflazacort, Desonide, Desoximethasone, Dexamethasone, Diflorasone, Diflucortolone, Difluprednate, Fluazacort, Flucoronide, Flumethasone, Flunisolide, Fluocinolone acetonide, Flucinonide, Fluocortin butyl, Fluocortolone, Fluorometholone, Fluperolone acetate, Fluprednilene acetate, Fluprednisolone, Flurandrenolide, Formocortal, Halcinonide, Halobetasol propionate, Halomatasone, Halopredone acetate, Hydrocortamate, Loteprednol etabonate, Medrysone, Meprednisone, Methylprednisolone, Mometasone furoate, Paramethasone, Prednicarbate, Prednisone, Prednisolone 21-diethylaminoacetate, Prednisolone sodium phosphate, Prednival, Prednylidene, Rimexolone, Triamcinolone, Triamcinolone acetonide, 21-Acetoxypregnenolone, Cortivazol, Amcinonide, Fluticasone propionate, Mazipredone, Tixocortol, Triamcinolone hexacetonide, Ursodeoxycholic acid, Chenodeoxycholic, Mytatrienediol, Ethynil Estradiol, Estradiol, Mestranol.

Non limiting examples of antitumoral drugs are:
Antacitabine, Anthramycin, Azacitidine, 6-Azauridine, Carubicin, Chlorambucil, Chlorozotocin, Cytarabine, Daunomicin, Defosfamide, Denopterin, Doxifluridine, Doxorubicin, Droloxifene, Edatrexate, Eflornithine, Enocitabine, Epirubicin, Epitiostanol, Etanidazole, Etoposide, Fenretinide, Fludarabine, Fluorouracil, Gemcitabine, Hexestrol, Idarubicin, Lonidamine, Melphalan, 6-mercaptopurine, Methotrexate, Mitoxantrone, Mycophenolic acid, Pentostatin, Pirarubicin, Piritexim, Podophyllic acid, Puromycin, Retinoic acid, Roquinimex, Streptonigrin, Teniposide, Tenuazonic acid, Thiamiprine, Thioguanine, Tomudex, Topotecan, Trimetrexate, Tubercidin, Ubenimex, Zorubicin.

Non limiting examples of β-adrenergic compounds are:
Albuterol, Bambuterol, Bitoterol, Carbuterol, Clenbuterol, Chlorprenalina, Dioxethedrine, Ephedrine, Epinephrine, Etafredine, Ethylnorepinephrine, Fenoterol, Isoetharine, Isoprotenerol, Mabuterol, Metaproterenol, Pirbuterol, Salmeterol, Soterenol, Terbutalina, Tuloterol, Procaterol, Bufetalol, Acebutolol, Alprenolol, Arotinolol, Atenolol, Betaxolol, Bevantolo, Bucumolol, bufuralol, Bunitrolol, Bupranolol, Carazolol, Carteolol, Celiprolol, Epanolol, Indenolol, Mepindolol, Metoprolol, Nadolol, Nifenalol, Penbutolol, Pindolol, Pronethalol, Propanolol, Sotalol, Timolol, Toliprolol, Butofilol, Cervedilol, Cetamolol, Dilevalol, Esmolol, Labetalol, Metipranolol, Moprolol, Nebivolol, Oxprenolol, Practolol, Sulfinalol, Tertatolol, Tilisolol, Xibenolol, Eprozinol, Etophylline, Exoprenaline, Propoxyphilline, Reproterol, Rimiterol, 1-Teobrominacetic acid, Tetroquinol, Nadoxolol.

Non limiting examples of antihyperlipoproteinemic compounds are:
Atovarstatin, Cilastatin, Dermostatin A, Dermostatin B, Fluvastatin, Lovastatin, Mevastatin, Nystatin A₁, Pentostatin, Pepstatin, Sinvastatin

Non limiting examples of bone resorption inhibitors are:
Alendronic acid, Butedronic acid, Etidronic acid, Oxidronic acid, Pamidronic acid, Risedronic acid.
The chemical formula of the above listed compounds is reported on the Merck Index, Twelfth Edition.
Preferred drugs useful in the present invention are selected form the following formulas: 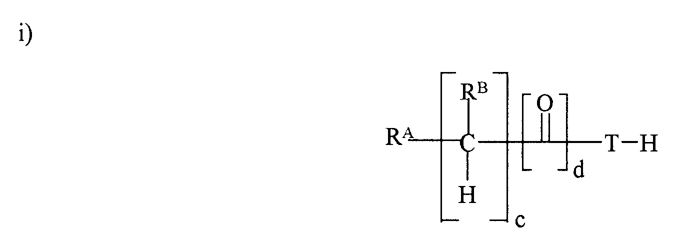 where
**c** and **d** are independently 0 or 1;
**T** is selected from the group consisting of O, NH and S;
**R**^{**B**} is selected from the group consisting of H, a linear or branched C₁-C₁₂ alkyl, C₂-C₁₂ alkenyl; preferably R^{B} is H, an alkyl having from 1 to 4 carbon atoms, most preferably R^{B} is CH₃
When **c** is equal to 0, **d** is 1, **R**^{**A**} is selected from the group consisting of: 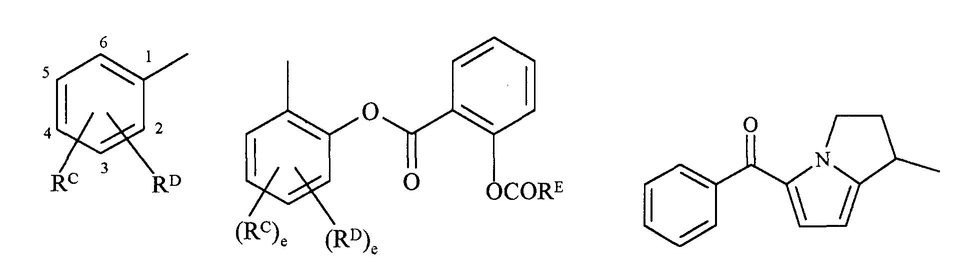 wherein:
**R**^{**C**} is selected from the group consisting of amino, R^{E}CONH-, OCOR^{E} group, and the residue of a heterocycle with a single ring having 5 or 6 atoms which may be aromatic, partially or totally hydrogenated, containing one or more heteroatoms independently selected from the group consisting of O, N, and S;
**R**^{**E**} is selected from the group consisting of methyl, ethyl and a linear or branched C₃-C₅ alkyl;
**R**^{**D**} is H, OH, halogen, a linear or when permissible branched alkyl having 1 to 4 atoms, a linear or when permissible branched alkoxyl having 1 to 4 atoms, a linear or when permissible branched perfluoroalkyl having 1 to 4 carbon atoms, for example trifluoromethyl, amino, mono- or di-(C₁-C₄) alkylamino;
**e** is 0 or 1;
when **c** is equal to 1, **d** is equal to 1, **R**^{**B**} is hydrogen, **R**^{**A**} is selected from the group consisting of: 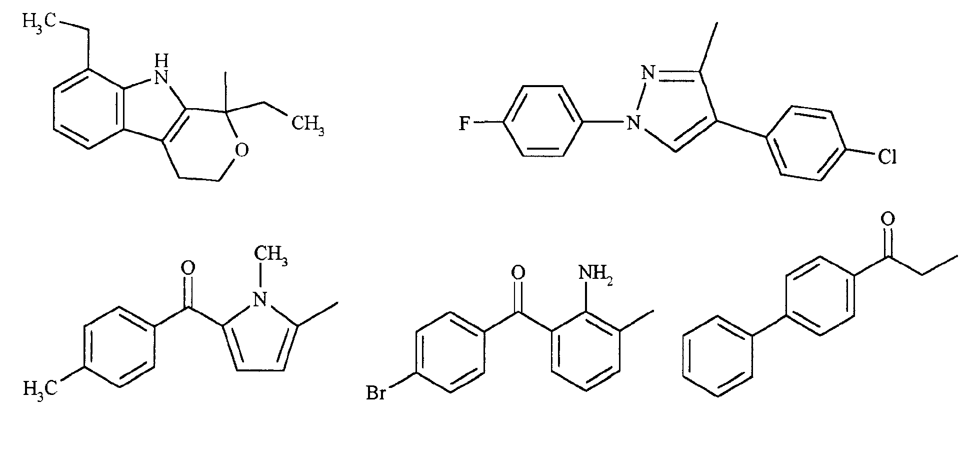 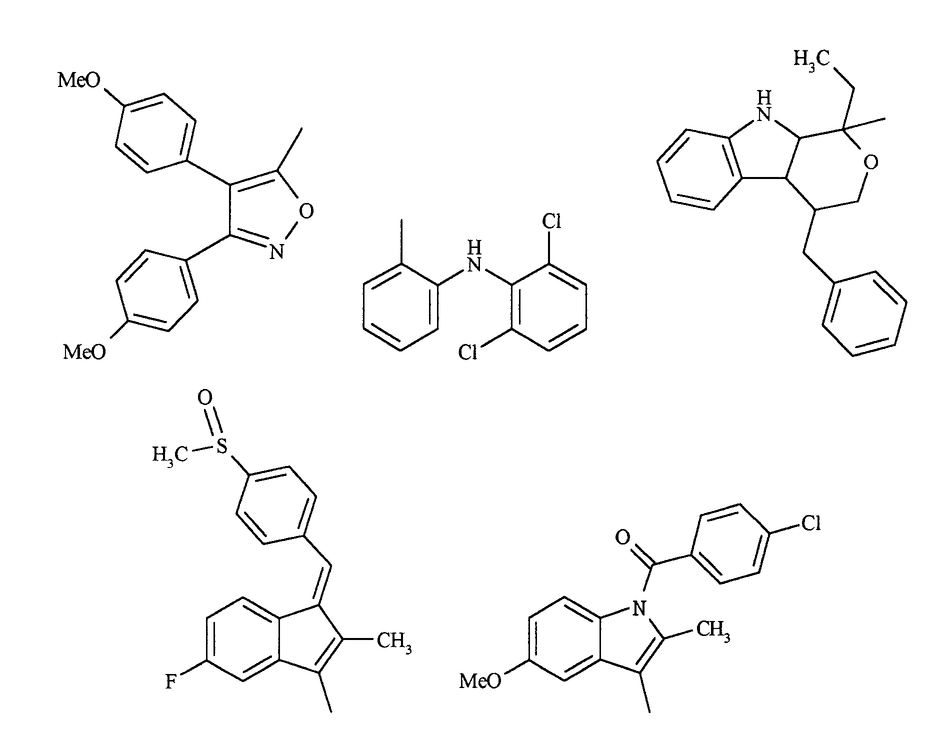 when **c** is equal to 1, **d** is equal to 1 and **R**^{**B**} is CH₃, **R**^{**A**} is selected from the group consisting of: 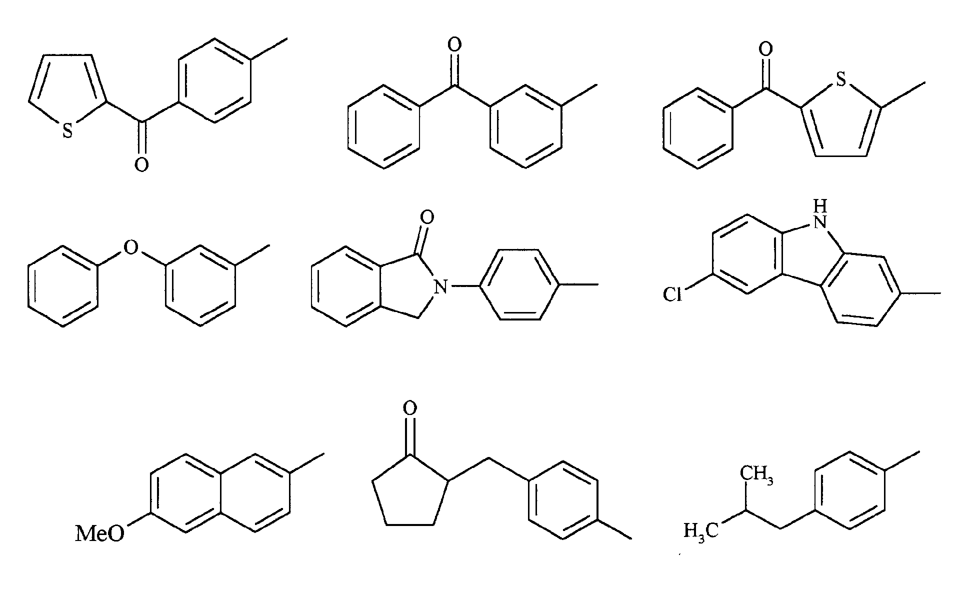 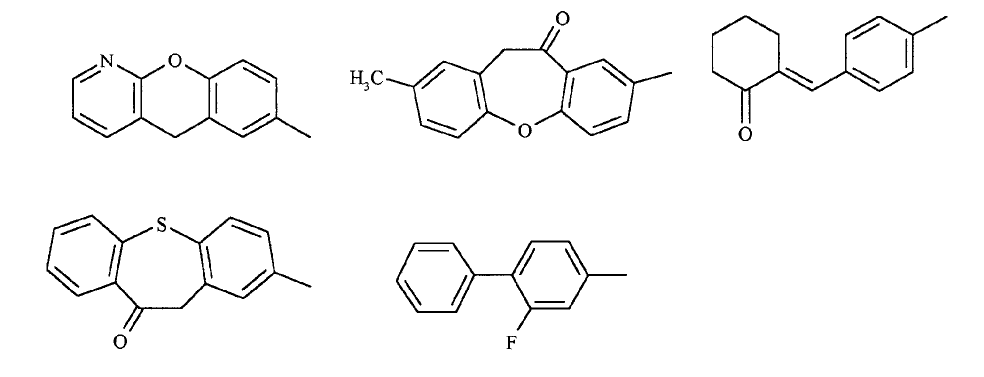 when **c** is equal to 0, **d** is equal to 0, **R**^{**A**} is selected from the group consisting of: 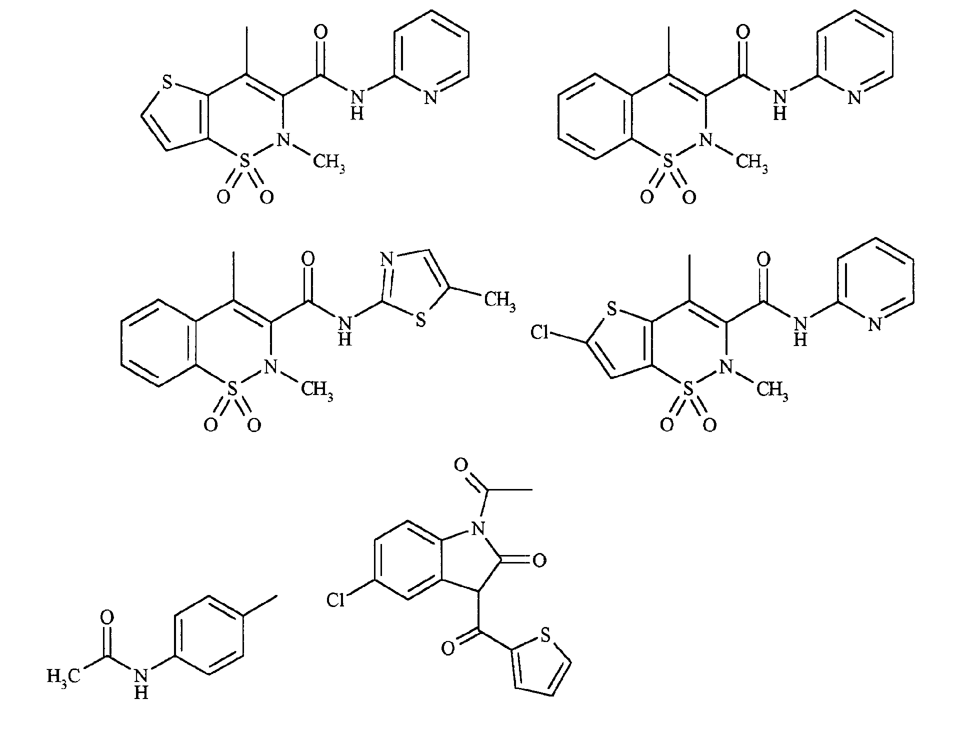 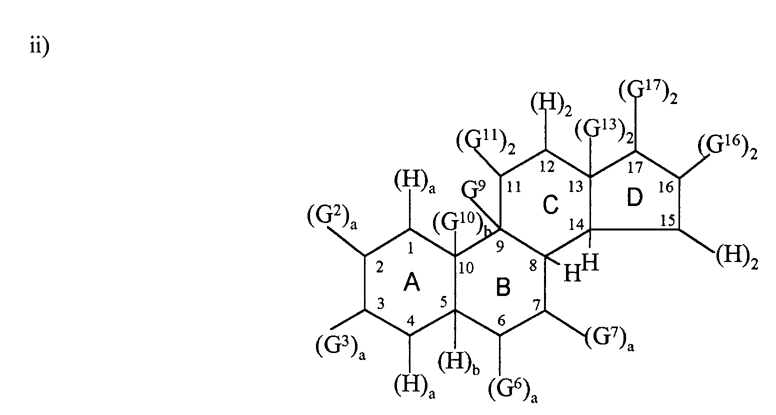 wherein:
at the position 1-2, 2-3, 3-4, 4-5, 5-6, 6-7, 5-10 there may be a double bond; the ring A is optionally an aromatic ring;
**a** is equal to 1 or 2, **b** is equal to 0 or 1;
each **G**^{**2**} is independently selected from the group consisting of H, Cl, Br;
each **G**^{**3**} is independently selected from the group consisting of H, O-CH₃, O-CH₂-CH₂-Cl, OH; two G³ can form a carbonyl group with the C³ atom;
one G² and one G³ can unite to form a ring of formula 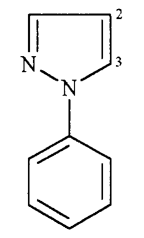
wherein
C²=C³ are part of the steroid structure;
each **G**^{**6**} is independently selected from the group consisting of H, Cl, F, CH₃, -CHO;
each **G**^{**7**} is independently selected from the group consisting of H, Cl, OH;
each **G**^{**9**} is independently selected from the group consisting of H, Cl, F;
**G**^{**10**} is selected from the group consisting of H, Cl, F, CH₃, -CHO;
each **G**^{**11**} is independently selected from the group consisting of H, OH, , Cl; two G¹¹ can form a carbonyl group with the C¹¹ atom;
each **G**^{**13**} is independently selected from the group consisting of H, CH₃;
each **G**^{**16**} is independently selected from the group consisting of H, CH₃, OH; two G¹⁶ can form a vinyl group with the C¹⁶ atom;
each **G**^{**17**} is independently selected from the group consisting of H, OH and a monovalent radical comprising from 1 to 20 carbon atoms and from 0 to 5 oxygen, sulfur, nitrogen, halogen atoms; preferably it is H, OH, CH₃, C≡CH, CO-R-OH, CO-RH, CO-R-Cl, OCO-RH, CO-COO-RH, R-COOH, CH(OH)R-OH, COO-R-Cl, OC(O)O-RH, CO-R-SH, CO-R-O-CO-R-N(CH₂CH₃)₂, CO-SCH₂F, CO-R-OCORH, 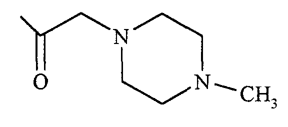 wherein R is a C₁-C₂₀ linear or branched alkylene radical, and 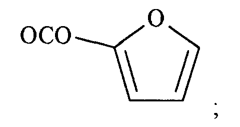
two G¹⁷ can form a carbonyl group with the C¹⁷ atom;
one G¹⁶ can unite with a G¹⁷ group to form, together with C¹⁶ and C¹⁷ the following groups: 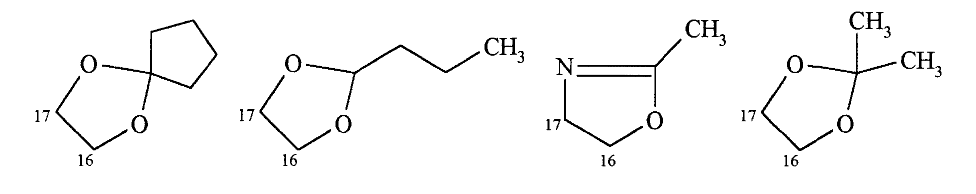 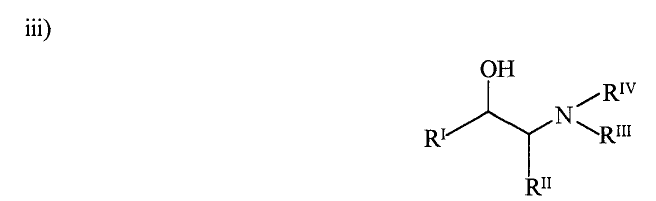
**R**^{**I**} is monovalent radical comprising from 6 to 20 carbon atoms and from 0 to 6 heteroatoms selected from oxygen, nitrogen, sulfur, chlorine, bromine, fluorine; examples of functional groups which are present in the radical R^{I} are the following: phenoxy, phenyl, thiazolyl, quinol-5-on-yl, pyridyl, tiofuranyl, tetrahydrofuranyl;
**R**^{**II**} is selected from the group consisting of hydrogen and linear or branched alkyl having from 1 to 4 carbon atoms, preferably R^{II} is selected from the group consisting of H, CH₃ and CH₃CH₂
**R**^{**III**} is selected from the group consisting of hydrogen and linear or branched alkyl having from 1 to 4 carbon atoms, preferably R^{III} is selected from the group consisting of H and CH₃;
**R**^{**IV**} is selected from the group consisting of hydrogen, a linear or branched alkyl having from 1 to 4 carbon atoms and a substituted aryl; preferably R^{IV} is selected from the group consisting of tert-butyl and isopropyl; 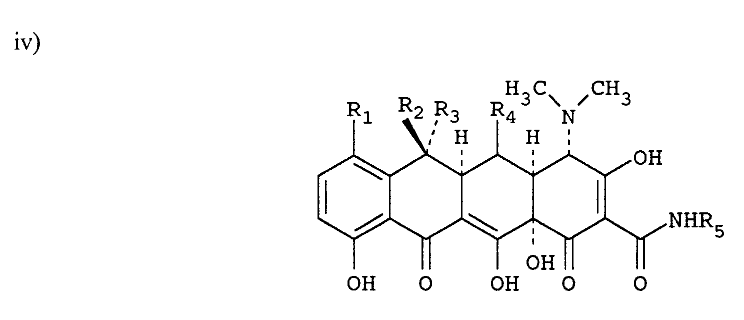 wherein:
   **R**_{**1**} is selected from the group consisting of H, Cl and dimethylamino,
   **R**_{**2**} is selected from the group consisting of H, OH,
   **R**_{**3**} is selected from the group consisting of H, CH₃,
   R₂ and R₃ together can be a methylene group (CH₂=),
   **R**_{**4**} is selected from the group consisting of H, OH,
   **R**_{**5**} is selected from the group consisting of H, CH₂OH and a monovalent radical containing from 5 to 20 carbon atoms and from 1 to 8 nitrogen atoms; the radical can further comprise other functional groups such as carboxyl and hydroxyl. 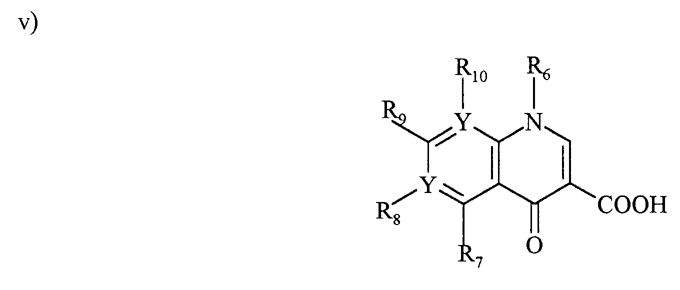
   wherein
   each **Y** is independently selected from the group consisting of C and N,
   **R**_{**6**} is selected from the group consisting of cyclopropyl, phenyl, 4-fluorophenyl, 2,4-difluorophenyl, 2-fluoroethyl and ethyl;
   **R**_{**7**} is selected from the group consisting of H, amino, methyl,
   **R**_{**8**} is selected from the group consisting of H and F;
   **R**_{**9**} is selected from the group consisting of H, methyl and a monovalent radical containing from 1 to 20 carbon atoms and from 1 to 4 nitrogen atoms;
   **R**_{**10**} is selected from the group consisting of H, Cl and F;
   R₆ e R₁₀ can unite to form an optionally substituted six membered ring optionally containing up to two heteroatoms selected from the group consisting of oxygen and sulfur: 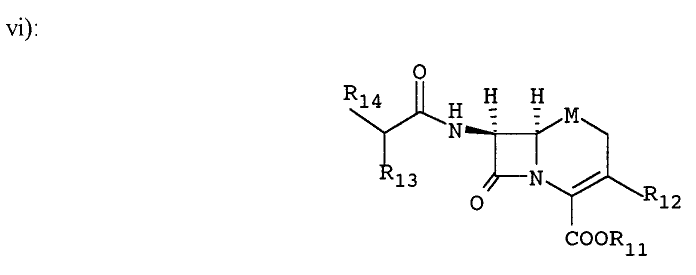
   wherein
   **M** is selected from the group consisting of sulfur, carbon or oxygen;
   **R**_{**11**} is selected from the group consisting of H, pivaloyloxymethyl,
   **R**_{**12**} is selected from the group consisting of chlorine and a monovalent radical containing from 1 to 5 carbon atoms, from 0 to 5 nitrogen atoms and from 0 to 1 sulfur atoms; preferably it is selected from chlorine, methyl, acetyloxymethyl, 2-propenyl 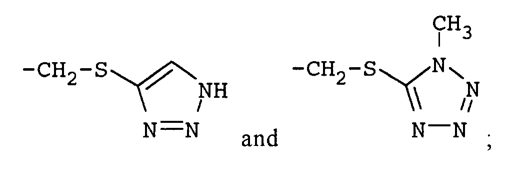
   **R**_{**13**} is selected from the group consisting of amino, hydroxyl and monovalent radical containing from 1 to 10 carbon atoms, from 0 to 5 oxygen atoms and from 0 to 5 nitrogen atoms;preferably it is selected from the group consisting of amino, hydroxyl, carboxyl and 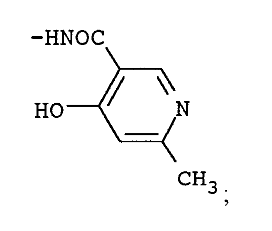
   **R**_{**14**} is an unsaturated C₆ ring, optionally substituted; preferably it is selected from the group consisting of phenyl, 1,4-cyclohexadienyl and 4-hydroxyphenyl. 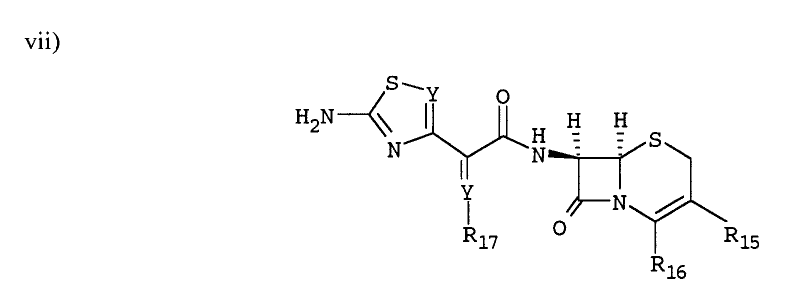
   wherein:
   each **Y** is independently selected from the group consisting of carbon and nitrogen
   **R**_{**15**} is selected from the group consisting of hydrogen and a monovalent radical containing from 1 to 12 carbon atoms, from 0 to 4 oxygen atoms, from 0 to 5 nitrogen atoms and from 0 to 3 sulfur atoms; preferably it is selected from the group consisting of H, methyl, ethyl, ethenyl, NH₂COOCH₂-, CH₃COOCH₂-, pyridilmethylene and 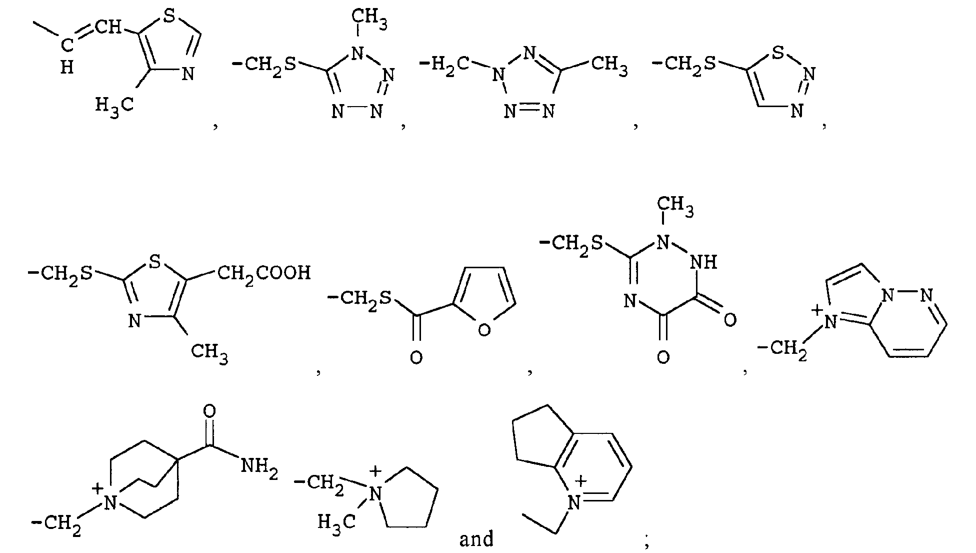
   **R**_{**16**} is a monovalent radical containing from 1 to 10 carbon atoms and from 2 to 8 oxygen atoms; preferably it is selected from the group consisting of carboxyl, (CH₃)₃CCOOCH₂OCO- and (CH₃)₂CHOCOOCH(CH₃)OCO-; when R₁₅ is a quaternary ammonium cation, R₁₆ is optionally a -COO⁻;
   **R**_{**17**} is selected from the group consisting of -OH and a monovalent radical containing from 1 to 12 carbon atoms and from 0 to 4 oxygen atoms, preferably it is selected from the group consisting of -OH, -OCH₃, -CH₂CH₃, -OCH₂COOH, -CH₂COOH, OC(CH₂)₃-COOH. 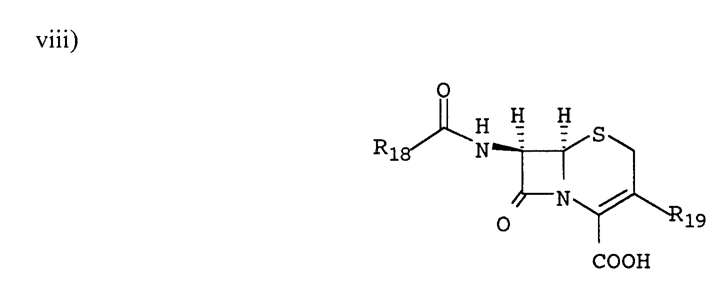
   wherein:
   **R**_{**18**} is a monovalent radical containing from 1 to 20 carbon atoms, from 0 to 4 oxygen atoms, from 0 to 5 nitrogen atoms, from 0 to 3 sulfur atoms and from 0 to 3 chlorine atoms; preferably it is selected from the group consisting of: PhCH(OH)-, -CH₂CN 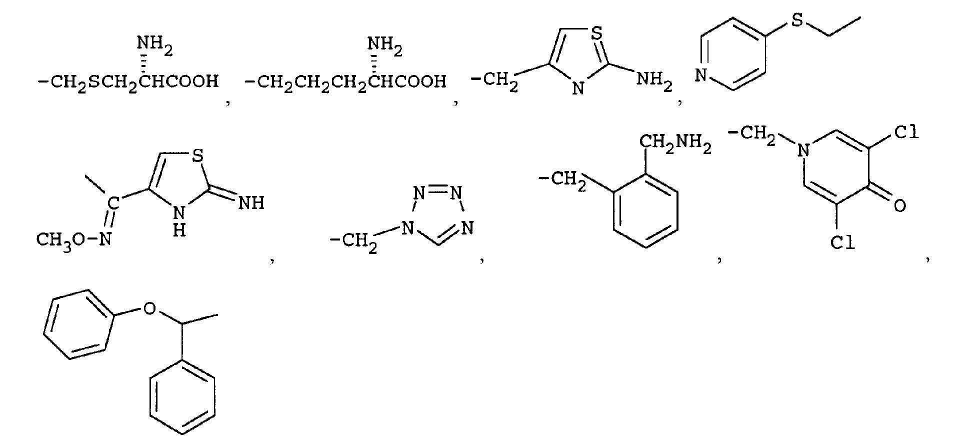
   **R**_{**19**} is selected from the group consisting of H and a monovalent radical containing from 1 to 10 carbon atoms, from 0 to 4 oxygen atoms, from 0 to 6 nitrogen atoms and from 0 to 3 sulfur atoms; preferably it is selected from the group consisting of: CH₃COOCH_{2,} 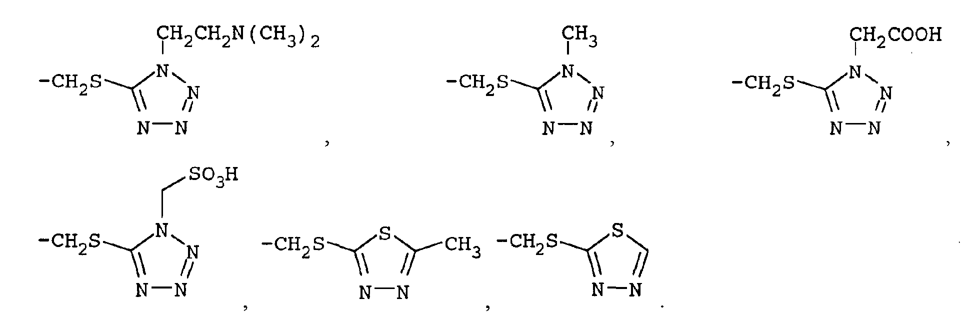 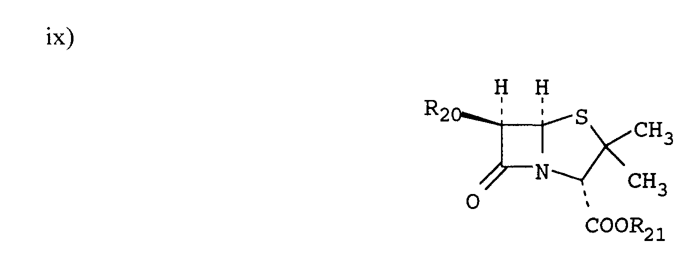
wherein:
**R**_{**20**} is a monovalent radical containing from 1 to 20 carbon atoms, from 0 to 8 oxygen atoms, from 0 to 5 nitrogen atoms, from 0 to 3 sulfur atoms, from 0 to 3 fluorine atoms and from 0 to 3 chlorine atoms; preferably it is selected from the group consisting of: 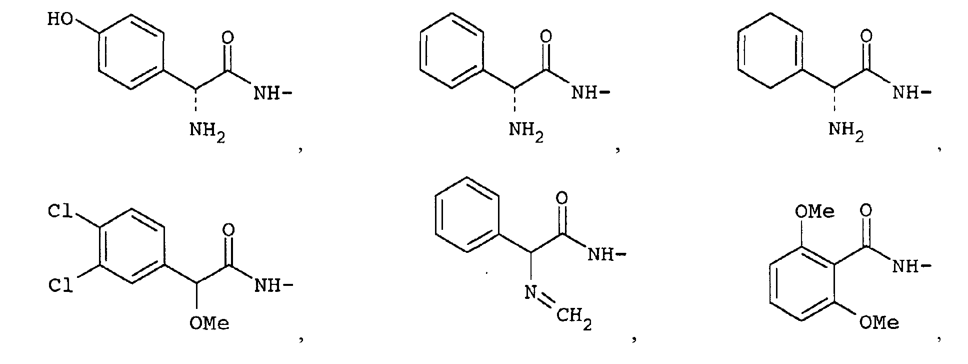 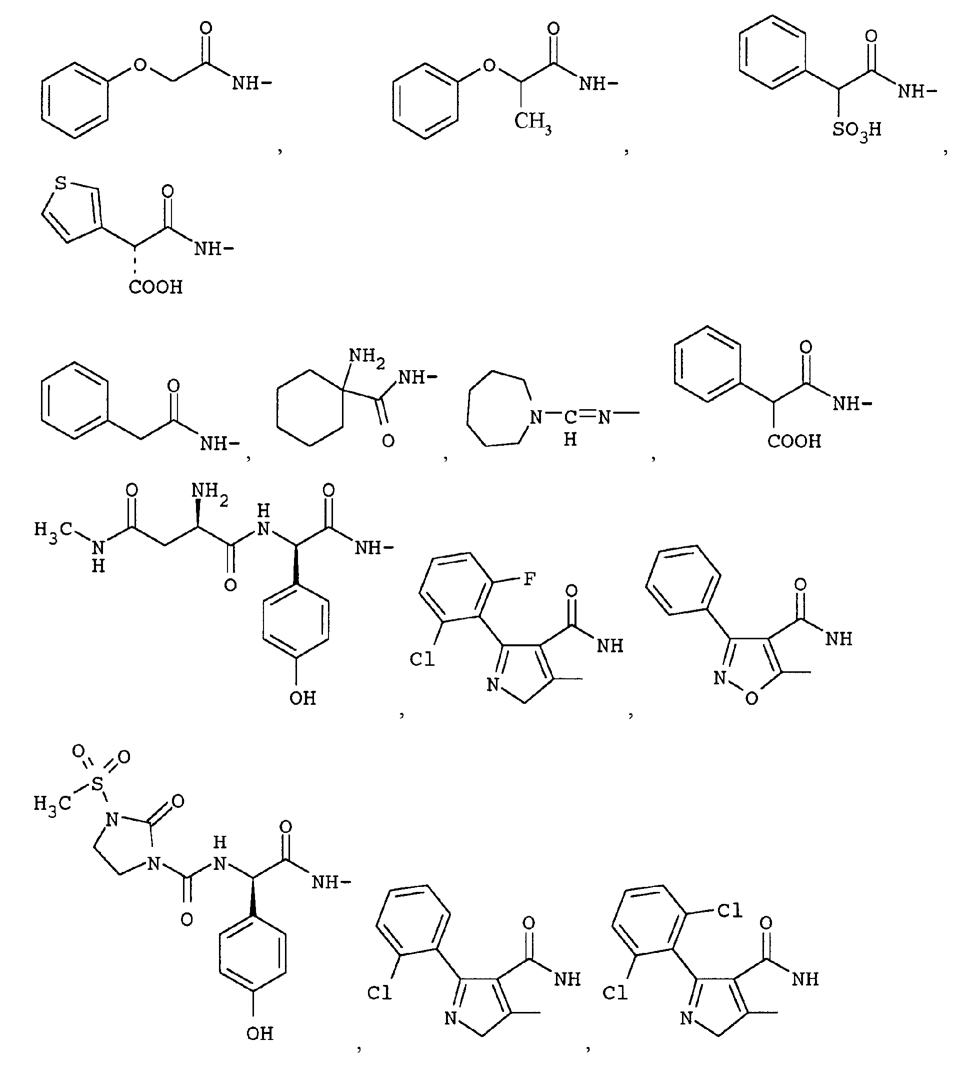 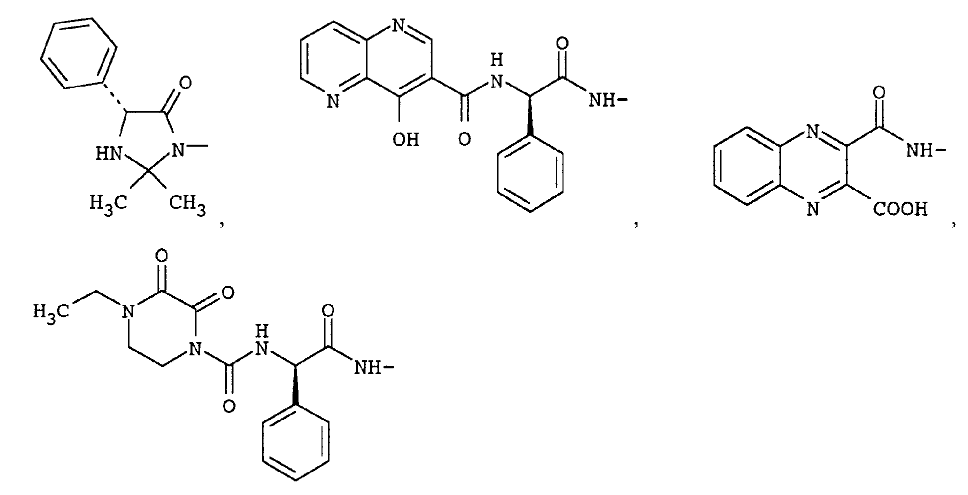 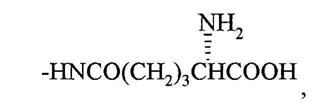 -NHCO(CH₂)₃CH(NH₂)COOH, CH₂=CH₂SCH₂CONH-;
**R**_{**21**} is selected from the group consisting of H and a monovalent radical containing from 1 to 20 carbon atoms, from 0 to 8 oxygen atoms, from 0 to 5 nitrogen atoms and from 0 to 3 sulfur atoms; preferably it is selected from the group consisting of: H, -CH₂OCOC(CH₃)₃, -CH(CH₃)OCOOC₂H₅, -CH₂CH₂N(CH₂CH₃)₂, 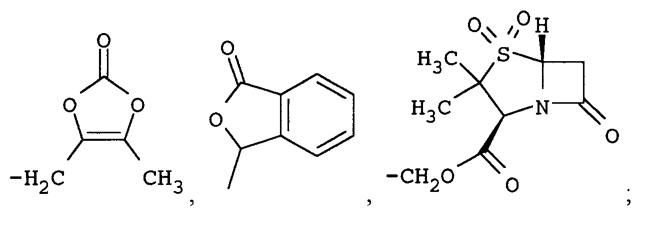 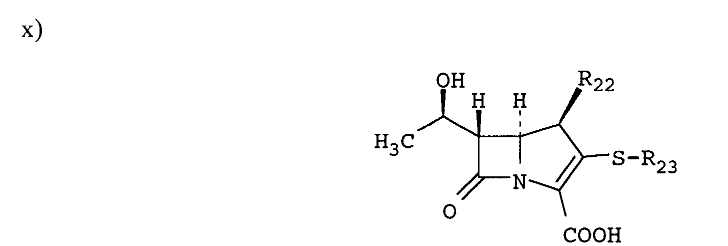
wherein:
**R**_{**22**} is selected from the group consisting of H and methyl;
**R**_{**23**} a monovalent radical containing from 1 to 10 carbon atoms, from 0 to 4 oxygen atoms and from 1 to 5 nitrogen atoms; preferably it is selected from the group consisting of: -CH₂CH₂NHCH=NH, 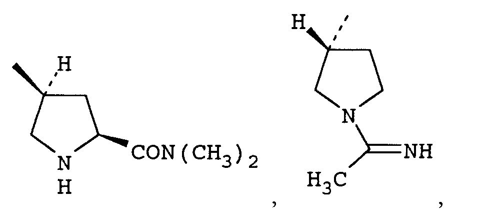 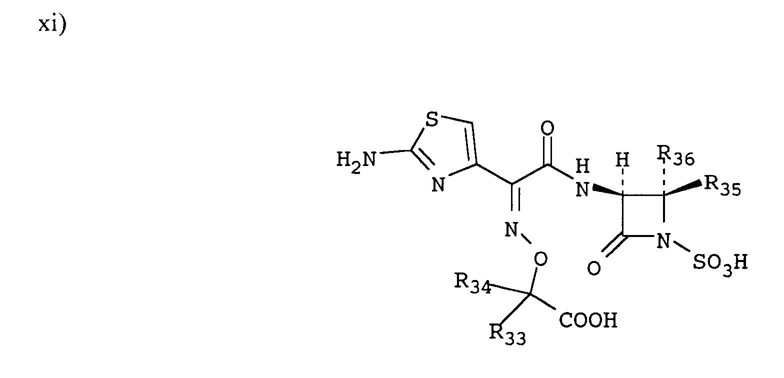
wherein:
R₃₃ , R₃₄ and R₃₆ are independently selected from the group consisting of H and CH₃;
R₃₅ is selected from the group consisting of H and -CH₂OCONH₂, 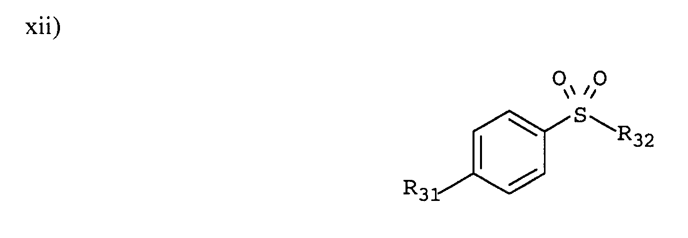
wherein:
**R**_{**31**} is selected from the group consisting of -NH₂, -CH₂NH₂ and -NHCH₂Ph
**R**_{**32**} is selected from the group consisting of -NH₂, -NHR₂₆ and a monovalent radical containing from 1 to 20 carbon atoms, from 0 to 5 oxygen atoms, from 0 to 5 nitrogen atoms and from 0 to 3 sulfur atoms; wherein **R**_{**26**} is a monovalent radical containing from 1 to 20 carbon atoms, from 0 to 8 oxygen atoms, from 0 to 5 nitrogen atoms, from 0 to 3 sulfur atoms and from 0 to 3 chlorine atoms;
preferably **R**_{**32**} is selected from the group consisting of: 4-(2-hydroxyethylamino)phenyl, guanyl, 4-(amino)phenyl, 4-(aminomethyl)phenyl, 4-(carboxymethylamino)phenyl, succinylaminophenyl, 2-amino-5-thiazolyl;
preferred examples of R₂₆ are: acetyl, carbamoyl, 3-methyl-2-butenoyl, aminothioxomethylene, 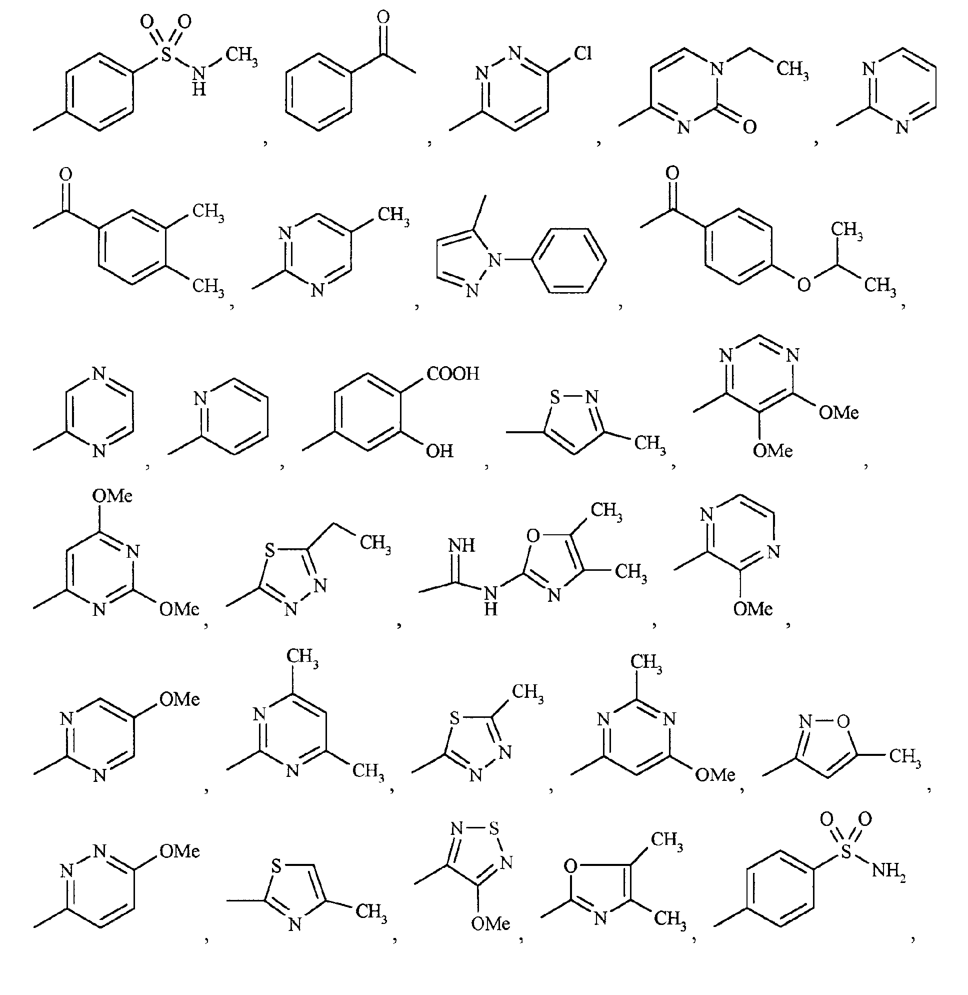 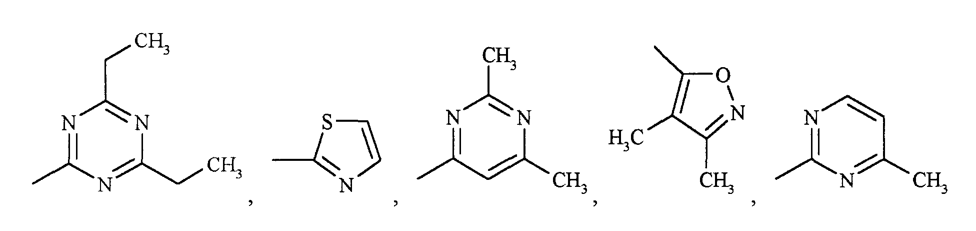 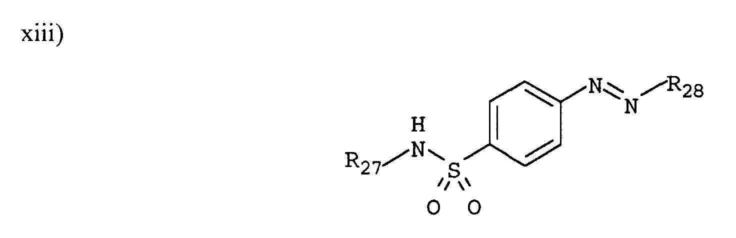 wherein:
R₂₇ is selected from the group consisting of H and 4,6-dimethyl-2-pyrimidinyl;
R₂₈ is a phenyl group substituted in at least 2 of the positions 2, 3, 4 and 6 by a group selected from hydroxyl, carboxyl and amino; preferred examples of R₂₈ are 2,4-diamino-6-carboxyphenyl, 2,4-diaminophenyl, 3-carboxy-4-hydroxyphenyl; 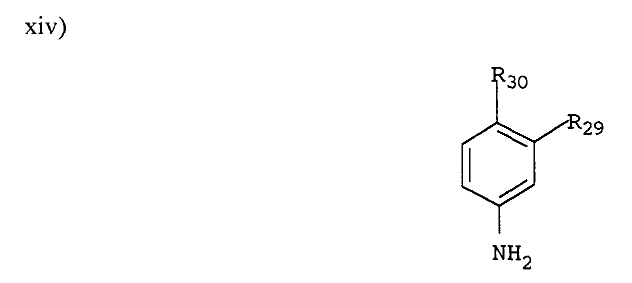
wherein:
**R**_{**29**} is selected from the group consisting of hydrogen and hydroxyl
**R**_{**30**} is selected from the group consisting of carboxyl, phenoxycarbonyl, 4-(amino)phenylsulfinyl, hydrazinocarbonyl; 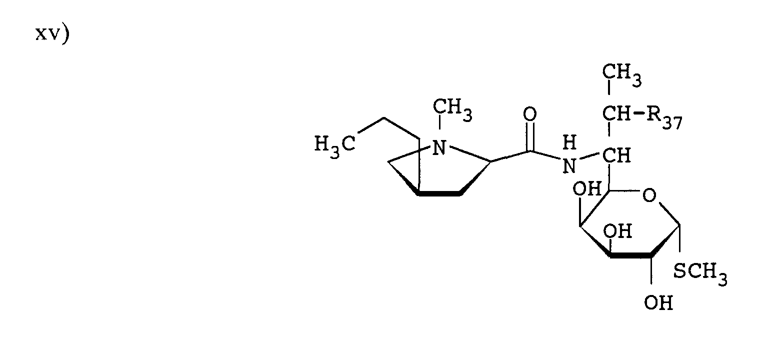
wherein:
**R**_{**37**} is selected from the group consisting of Cl and -OH; 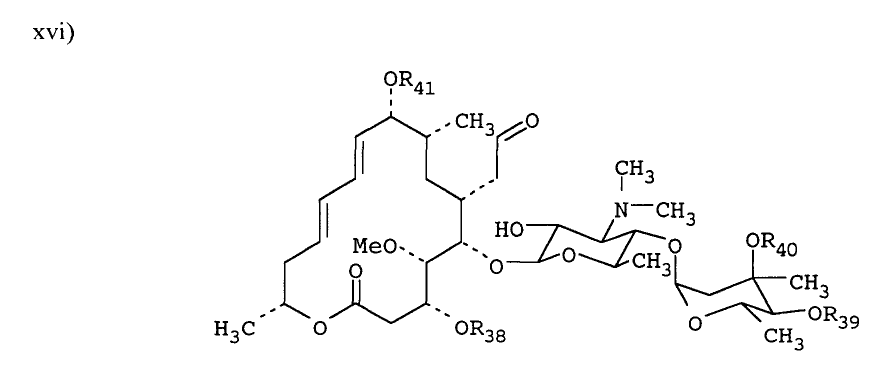
wherein:
**R**_{**38**} **R**_{**39**} **R**_{**40**} are independently selected from the group consisting of H and acyl; preferably they are selected from the group consisting of H acetyl, propionyl, butyrryl, valeryl
**R**_{**41**} is independently selected from the group consisting of H and 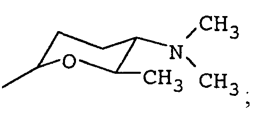 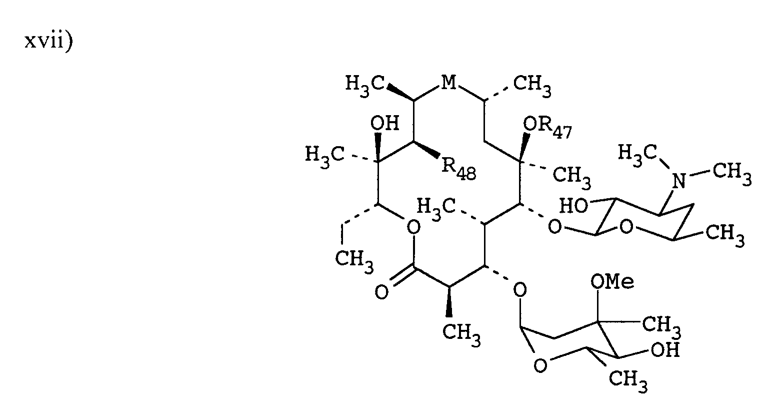
wherein:
**R**_{**47**} is selected from the group consisting of H and -CH₃
**M** is selected from the group consisting of CO, N-methyl-aminomethylene and -CH(NHR₄₉)- wherein **R**_{**49**} is a substituted methylene bridge connecting N with **R**_{**48**}
**R**_{**48**} is hydroxyl or, when **M** is -CH(NHR₄₉)-, is -O-;

Preferably **R**_{**49**} is 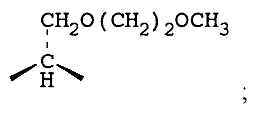 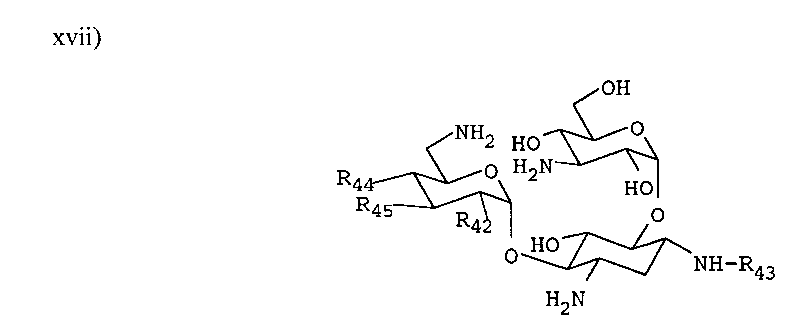 wherein:
**R**_{**42**} is selected from the group consisting of hydroxyl and amino;
**R**_{**43**} is selected from the group consisting of hydrogen, (R) and (S)-4-amino-2-hydroxybutyrryl
**R**_{**44**} and **R**_{**45**} are independently selected from the group consisting of hydrogen and hydroxyl. 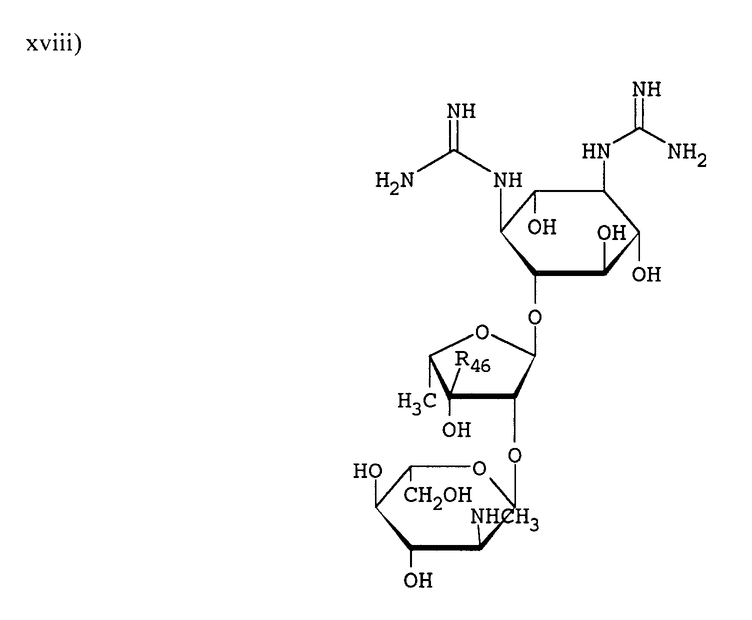
wherein:
**R**_{**46**} is selected from the group consisting of -CH₂OH and -CHO; 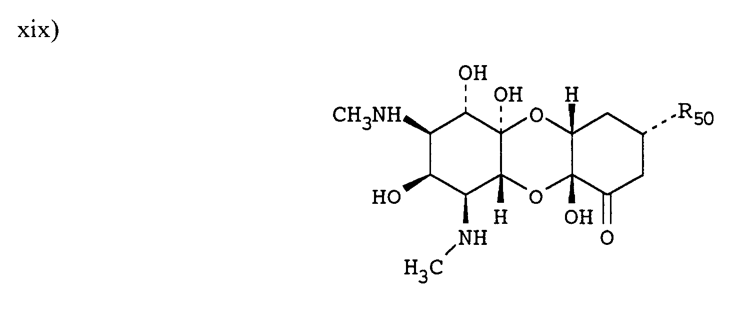
wherein:
**R**_{**50**} is a C₁-C₄ alkyl, preferably it is selected from the group consisting of methyl and n-butyl. 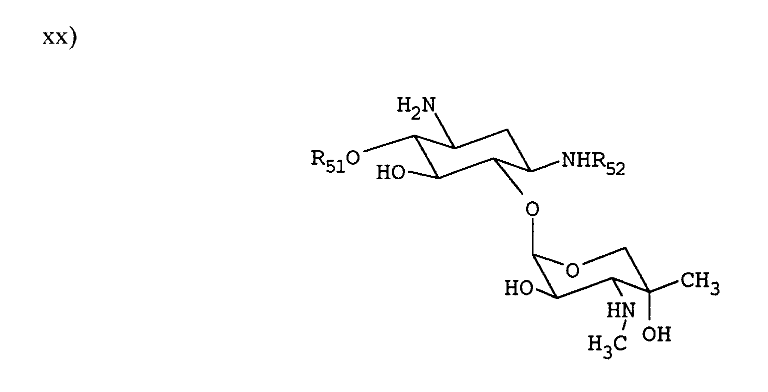
wherein:
**R**_{**51**} is independently selected from the group consisting of 3-amino-6-(aminomethyl)-3,4-dihydro-2H-pyran-2-yl and 2-amino-2,3,4,6-tetradeoxy-6-(methylamino)-α-D-eritrohexopyranosyl,
**R**_{**52**} is selected from the group consisting of H and -CH₂CH₃. 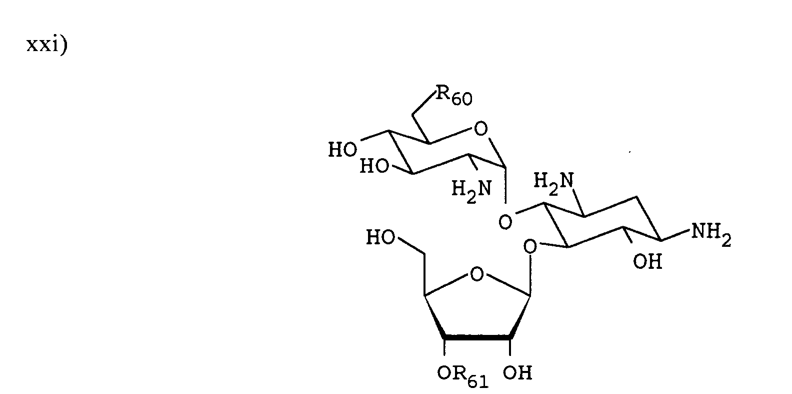
wherein:
**R**_{**60**} is selected from the group consisting of-OH and -NH₂;
**R**_{**61**} is selected from the group consisting of H, 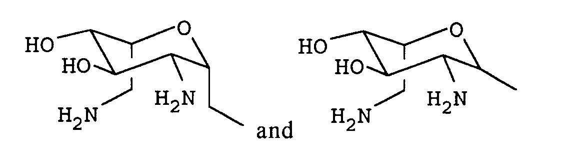 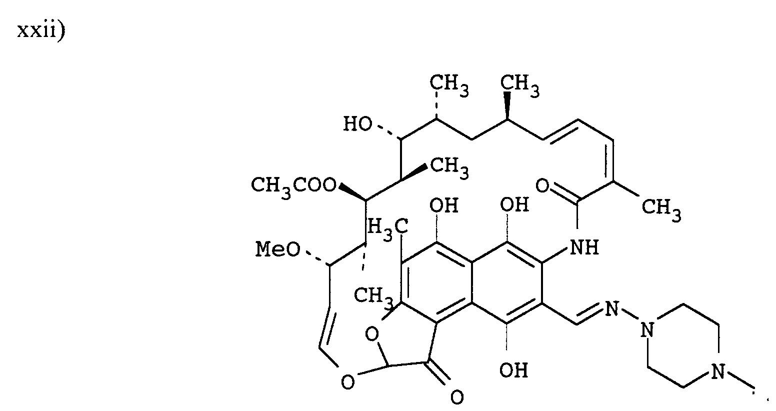
wherein **R**_{**54**} is a C₁-C₄ linear or cyclic alkyl, preferably it is selected from the group consisting of methyl and cyclopropyl.

In a preferred embodiment **X** is a divalent radical having the following structure : L'-X', wherein
**X'** is a divalent radical comprising from 1 to 50 carbon atoms, from 0 to 10 nitrogen atoms, from 0 to 20 oxygen atoms, from 0 to 2 sulfur atoms and from 0 to 8 halogen atoms.
**L'** is selected from the group consisting of O, S, NR' and CO; with **R'** selected from the group consisting of H and linear and branched C₁-C₄ alkyl;

In a preferred embodiment X' is represented by the following formula: 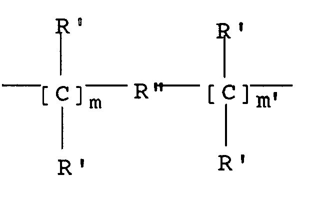 wherein:
**m** is selected from 0, 1, 2 and 3; preferably it is 1;
**m'** is selected from 1, 2 and 3; preferably it is 1;
each **R'** is independently selected from the group consisting of H, linear and branched C₁-C₄ alkyl; preferably it is H;
**R"** is selected from the group consisting of: 5 and 6 membered saturated, unsaturated and aromatic heterocycles, phenyl, optionally substituted by a carboxylic group;
When R" is an heterocycle, it is preferably selected from the group consisting of the following divalent radicals: 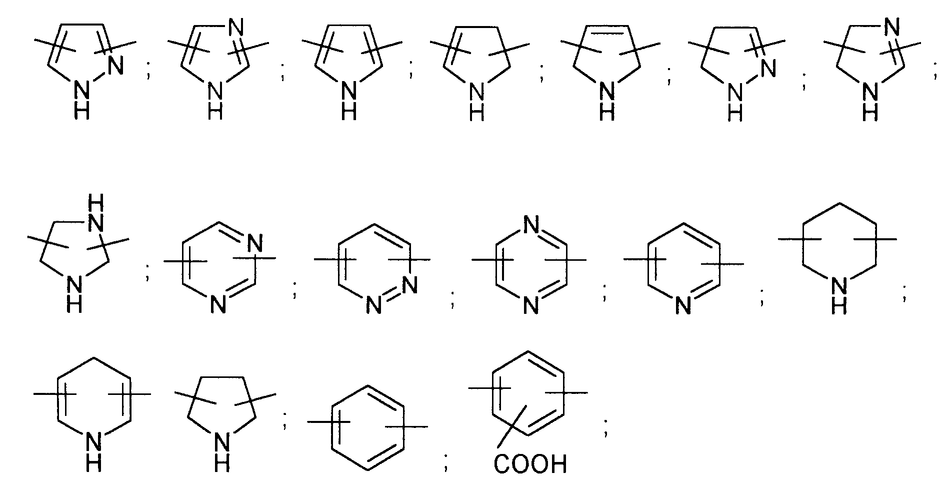 More preferably R" is selected from the group consisting of a pyridyl and pyrazolyl radical, most preferably it is selected from the group consisting of 2,3-, 2,6- pyridyl and 3, 5-pyrazolyl radicals, wherein 2, 3, 5 and 6 indicate the positions connecting the ring to the carbons of the bridge.
In another preferred embodiment X' is a C₁-C₂₀ alkylene group, preferably C₂-C₆, optionally substituted by - NH₂, -OH, NHCOR^{E} wherein R^{E} is selected from the group consisting of methyl, ethyl, linear or branched C₃-C₅ alkyl; a C₅-C₇ cycloalkylene group, optionally substituted by one or more C₁-C₆ alkyl chains;
In a third preferred embodiment X' is selected from the group consisting of a group of formula :

-CHR"'-CHR"'-(O-CHR"'-CHR"')ₚ- and -CHR"'-CHR"'-CHR"'-(O-CHR"'-CHR"'-CHR"')ₚ-

wherein each **R"'** is independently selected from the group consisting of H and CH₃
**p** varies from 1 to 6, preferably from 1 to 4.
In another preferred embodiment the group X comprises a radical having specific antioxidant properties as disclosed in WO 00/61537, WO 00/61541, WO 00/61604.
Non limiting examples of compounds from which the antioxidant radical is derived are: Aspartic acid, Histidine, 5-Hydroxytryptophan, 4-Thiazolidincarboxylic acid, 2-Oxo-4-thiazolidincarboxylic acid, 2-Thiouracil, 2-Mercaptoethanol, Esperitine, Secalciferol, 1-α-OH-vitamin D2, Flocalcitriol, 22-Oxacalcitriol, 24,28-Methylene-1α-hydroxyvitamin D2, 2-Mercaptoimidazol, Succinic acid, L-Carnosine, Anserine, Selenocysteine, Selenomethionine, Penicillamine, N-Acetylpenicillamine, Cysteine, N-acetyl-cysteine, Glutathione or its esters, Gallic acid, Ferulic acid, Gentisic acid, Citric acid, Caffeic acid, Hydrocaffeic acid, p-Coumaric acid, Vanillic acid, Chlorogenic acid, Kynurenic acid, Syringic acid, Nordihydroguaiaretic acid, Quercetin, Cathechin, Kaempferol, Sulphurethyne, Ascorbic acid, Isoascorbic acid, Hydroquinone, Gossypol, Reductic acid, Methoxyhydroquinone, Hydroxyhydroquinone, Propyl gallate, Saccharose, Vitamin E, Vitamin A, 8-Quinolol, 3-ter-Butyl-4-hydroxyanisole, 3-Hydroxyflavone, 3,5-ter-Butyl-p-hydroxytoluene, p-ter-Butyl-phenol, Timolol, Xibornol, 3,5-di-ter-Butyl-4-hydroxybenzyl-thioglycolate, 4'-Hydroxybutyranilide, Guaiacol, Tocol, Isoeugenol, Eugenol, Piperonyl alcohol, Allopurinol, Conyferyl alcohol, 4-Hydroxyphenetyl alcohol, p-Coumaric alcohol, Curcumin, N,N'-Diphenyl-p-phenylenediamine, Ethoxyquin, Thionine, Hydroxyurea, 3,3'-Thiodipronic acid, Fumaric acid, Dihydroxymaleic acid, Thioctic acid, 3,4-Methylendioxycinnamic acid, Piperonylic acid, N-Ethylendiethanolamine, Thiodiethylenglycol. The following are non-limiting example which illustrate the invention.

### Experimental

### Example 1

Male Guinea pigs (weighing 300 to 500 g) were killed by a blown on the neck and exsanguinated. Urinary bladders were cut into strip preparations (3x12 mm). Guinea-pig bladder strips were rapidly transferred to jacketed tissue baths (25 ml) and mounted between two hooks. One the hooks was connected to a force transducer (Gould UC2). The strips were maintained at 37°C in a physiological salt solution. (PSS) that contains the following components: NaCl (119 mM), KCl (4.6 mM), CaCl₂ (1.5 mM), MgCl₂ (1.2 mM), NaHCO₃ (20 mM), NaH₂PO₄ (1.4 mM) and glucose (11 mM).The solution was gassed with a 95/5 mixture of O₂/CO₂ until a pH of 7.4 was achieved. A tension of 0.5 g was initially applied to each preparation. During stabilization (40-60') the strips were repeatedly washed and the tension was adjusted. Tissue contraction was induced by corbachol 3x10⁻⁶ M.
The experiment compares the inhibition of contraction obtained by using a solution of the composition according to the invention with the effect achieved by the same drug without cyclodextrin. Both the composition and the drug were dissolved in dimethylsulphoxyde (DMSO) and then added to the tissue bath were the their concentration was 1.0x10⁻⁵ M.
The drug used is 2-fluoro-a-methyl[1,1'-biphenyl]-4-acetic acid 4-(nitrooxy) butyl ester (NO-1). F1 and F2 represent the following compositions:
F1: 1.340 g of α CD and 0.500 g of NO-1 mixed in in water and then dried.
F2: 1.820 g of dimethyl β CD and 0.500 g of NO-1 mixed in water and then dried.
F0 represents the comparative test performed by using NO-1 alone ( no CD).
The percentage of inhibition of contraction obtained were the following:

| Composition | Inhibition (%) |
|---|---|
| F1 | 26.05 |
| F2 | 31.52 |
| F0 (comparative) | 21.67 |

### Example 2

Male Guinea pigs (weighing 300 to 500 g) were killed by a blown on the neck and exsanguinated. The thoracic aorta artery was isolated, placed in a ice cold PPS that contains the following components: NaCl (119 mM), KCl (4.6 mM), CaCl₂ (1.5 mM), MgCl₂ (1.2 mM), NaHCO₃ (20 mM), NaH₂PO₄ (1.4 mM) and glucose (11 mM), cleaned of connective tissue and cut into transverse ring (3mm). Each ring was then suspended vertically in the organ chamber (25 ml) and mounted between two hooks in PPS maintained at 37°C and gassed with a mixture 95/5 of O₂/CO₂ until achievement of a pH 7.4. One of the hooks was connected to a force transducer (Gould UC2). A resting tension of 2 g was initially applied to each preparation. During stabilization (45') the strips are repeatedly washed and the resting tension is adjusted.
Aorta rings were precontacted with phenylephrine 3x10⁻⁶ M and exposed to the drug at a concentration 1.0x10⁻⁶ M.
The experiment compares the inhibition of contraction effect achieved by using a solution of the composition according to the invention with the effect achieved by the same drug without cyclodextrin. Both the composition and the drug were dissolved in dimethylsulphoxyde (DMSO).
The drug used is 2-(acetyloxy)benzoic acid 3-(nitrooxymethyl)phenyl ester (NO-2).
F1, F2 and F3 represent the following compositions:
F1: 1.470 g of α CD and 0.500 g of NO-2 mixed in water and then dried.
F2: 1.470 g of α CD and 0.500 g of NO-2 mixed in ethanol/water and then dri ed.
F3: 2.000 g of dimethyl β CD and 0.500 g of NO-2 mixed in water and then dried.
F0 represents the comparative test performed by using NO-2 alone ( no CD).
The percentages of inhibition obtained were the following:

| Composition | Inhibition (%) |
|---|---|
| F1 | 54 |
| F2 | 59 |
| F3 | 61 |
| F0 (comparative) | 19 |

## Claims

1. Composition comprising cyclodextrins and a NO-releasing drug of formula
A-X-L-NOₙ
wherein
**A** is the radical deriving from a drug;
**X** is a divalent radical connecting A with the NO-releasing group L-NOₙ;
**L** is selected from the group consisting of: O, S and NH;
**n** is 1 or 2.

2. Composition according to claim 1 wherein -L-NOₙ is -O-NO₂

3. Composition according to claims 1-2 wherein the cyclodextrin is selected from the group consisting of α CD, dimethyl α CD, trimethyl-α CD, β CD, dimethyl-β CD, trimethyl-β CD, 2-hydroxypropyl-β CD, 3-hydroxypropyl-β CD, 2,3-dihydroxypropyl-β CD, γ CD, dimethyl γ CD, trimethyl γ CD and polymeric CD.

4. Composition according to claim 1-3 wherein the drug is selected from the following compounds: non steroidal antiinflammatory and analgesic drugs, antibacterial (antibiotics), antiviral, steroids, antineoplastic, β-adrenergics (agonists and blockers), antihyperlipoproteinemic, bone resorption inhibitors.

5. Composition according to claim 1-4 wherein **X** is a divalent radical having the following structure: L'-X', wherein **X**' is a divalent radical comprising from 1 to 20 carbon atoms, from 0 to 5 nitrogen atoms, from 0 to 5 oxygen atoms, from 0 to 2 sulfur atoms and from 0 to 5 halogen atoms and **L'** is selected from the group consisting of O, S, NR', CO, with **R'** selected from the group consisting of H, linear and branched C₁-C₄ alkyl;

6. Composition according to claim 5 wherein X' is represented by the following formula: 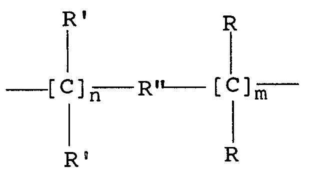 wherein:
**n** is selected from 0, 1, 2 and 3; preferably it is 1;
**m** is selected from 1, 2 and 3; preferably it is 1;
each **R'** is independently selected from the group consisting of H, linear and branched C₁-C₄ alkyl; preferably it is H;
**R"** is selected from the group consisting of: 5 and 6 membered saturated, unsaturated and aromatic heterocycles, phenyl, optionally substituted by a carboxylic group.

7. Composition according to claim 5 wherein X' is a C₁-C₂₀ alkylene group, preferably C₂-C₆, optionally substituted by - NH₂, -OH, NHCOR^{E} wherein R^{E} is selected from the group consisting of methyl, ethyl, linear or branched C₃-C₅ alkyl; a C₅-C₇ cycloalkylene group, optionally substituted by one or more C₁-C₆ alkyl chains;

8. Composition according to claim 5 wherein X' is selected from the group consisting of a group of formula :
-CHR"'-CHR"'-(O-CHR"'-CHR"')ₚ- and -CHR"'-CHR"'-CHR"'-(O-CHR"'-CHR"'-CHR"')ₚ-
wherein each **R"'** is independently selected from the group consisting of H and CH₃
**p** varies from 1 to 6, preferably from 1 to 4.

9. Composition according to claims 1-8 wherein the drug is selected form the following formulas 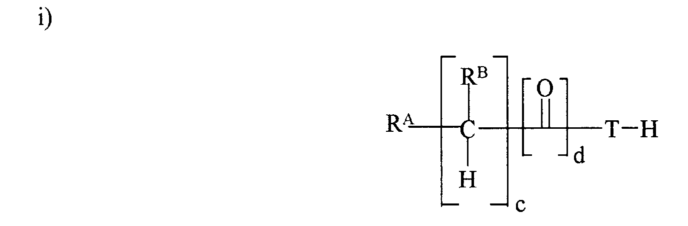 where
**c** and **d** are independently 0 or 1;
**T** is selected from the group consisting of: O, NH and S;
**R**^{**B**} is selected from the group consisting of H, a linear or branched C₁-C₁₂ alkyl, C₂-C₁₂ alkenyl;
When **c** is equal to 0, **d** is 1, **R**^{**A**} is selected from the group consisting of: 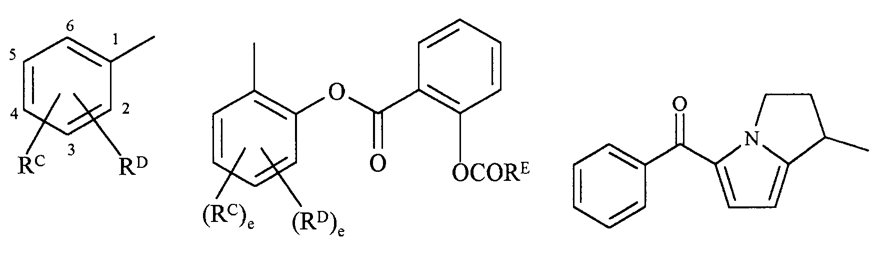 wherein:
**R**^{**C**} is selected from the group consisting of amino, R^{E}CONH-, OCOR^{E} group, and the residue of a heterocycle with a single ring having 5 or 6 atoms which may be aromatic, partially or totally hydrogenated, containing one or more heteroatoms independently selected from the group consisting of O, N, and S;
**R**^{**E**} is selected from the group consisting of methyl, ethyl and a linear or branched C₃-C₅ alkyl;
**R**^{**D**} is H, OH, halogen, a linear or when permissible branched alkyl having 1 to 4 atoms, a linear or when permissible branched alkoxyl having 1 to 4 atoms, a linear or when permissible branched perfluoroalkyl having 1 to 4 carbon atoms, for example trifluoromethyl, amino, mono- or di-(C₁-C₄) alkylamino;
**e** is 0 or 1;
when **c** is equal to 1, **d** is equal to 1, **R**^{**B**} is hydrogen, **R**^{**A**} is selected from the group consisting of: 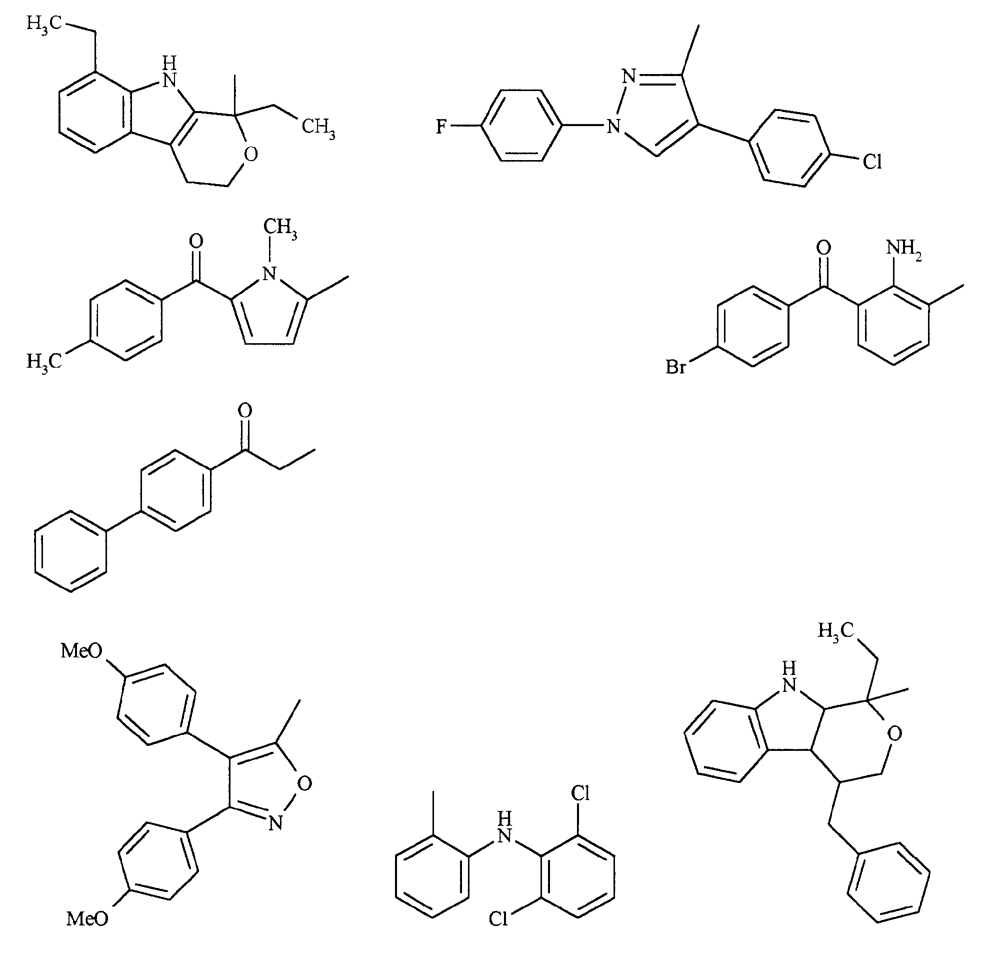 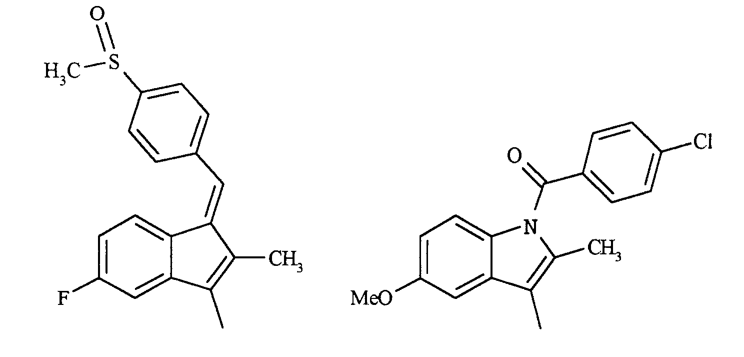 when **c** is equal to 1, **d** is equal to 1 and **R**^{**B**} is CH₃, **R**^{**A**} is selected from the group consisting of: 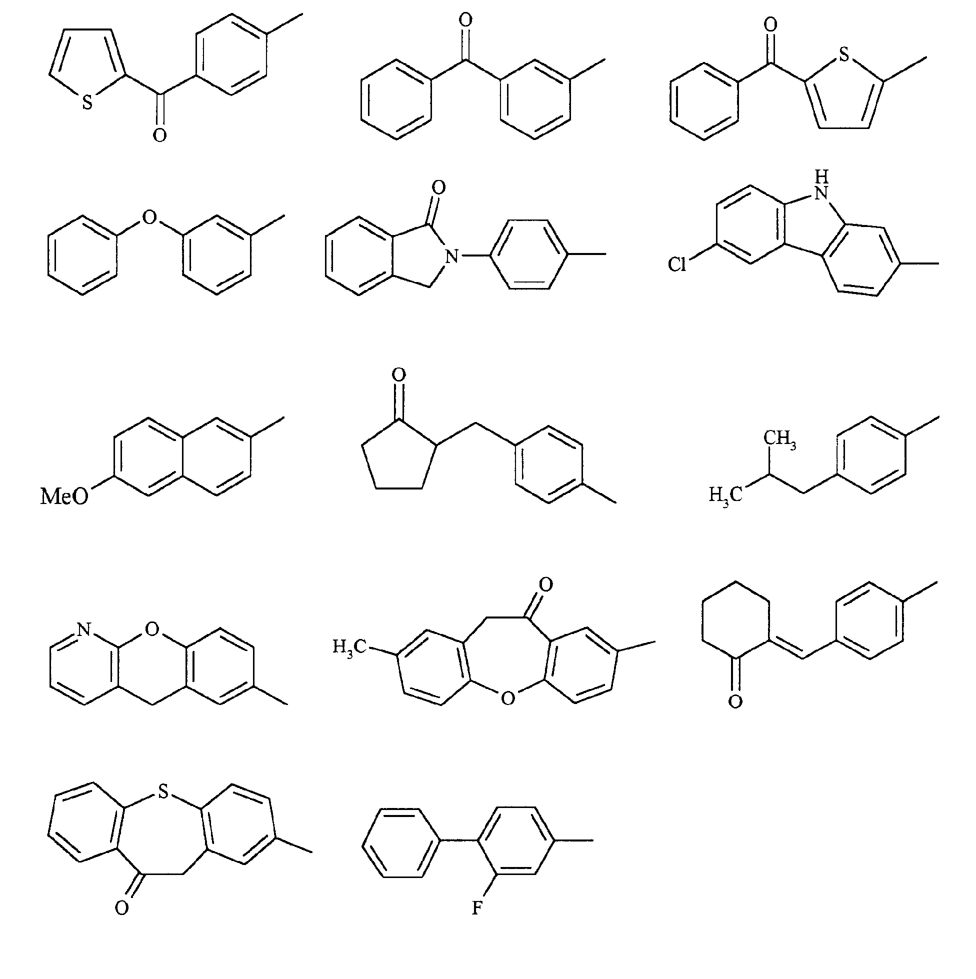 when **c** is equal to 0, **d** is equal to 0, **R**^{**A**} is selected from the group consisting of: 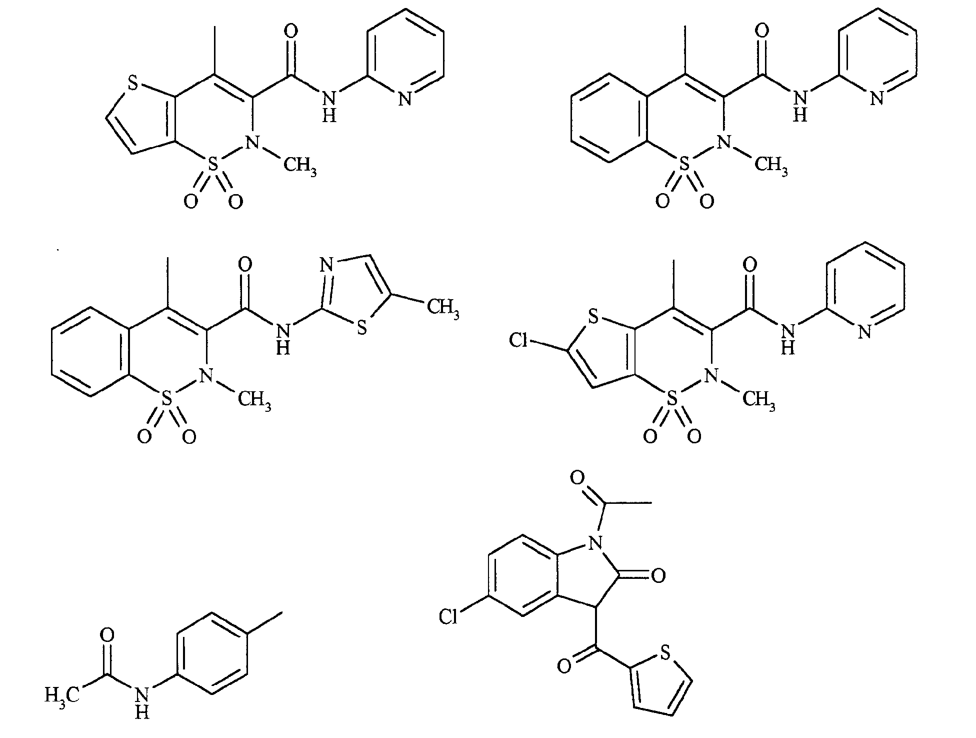 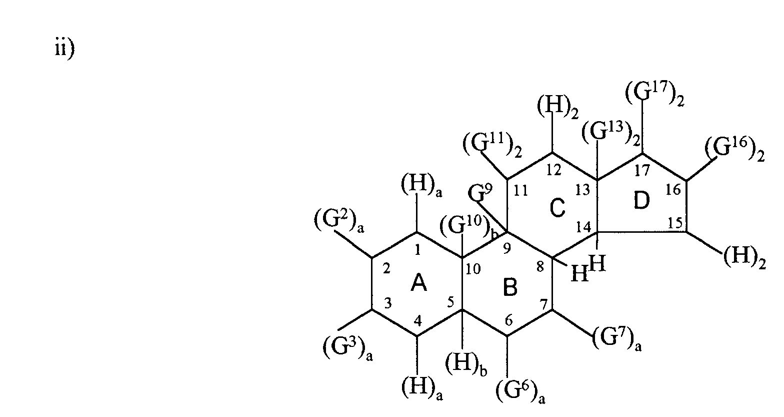 wherein:
at the position 1-2, 2-3, 3-4, 4-5, 5-6, 6-7, 5-10 there may be a double bond; the ring A is optionally an aromatic ring;
**a** is equal to 1 or 2, **b** is equal to 0 or 1;
each **G**^{**2**} is independently selected from the group consisting of H, Cl, Br;
each **G**^{**3**} is independently selected from the group consisting of H, O-CH₃, O-CH₂-CH₂-Cl, OH; two G³ can form a carbonyl group with the C³ atom;
one G² and one G³ can unite to form a ring of formula 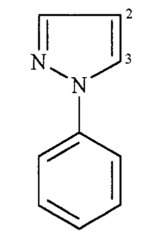
wherein
C²=C³ are part of the steroid structure;
each **G**^{**6**} is independently selected from the group consisting of H, Cl, F, CH₃, -CHO;
each **G**^{**7**} is independently selected from the group consisting of H, Cl, OH;
each **G**^{**9**} is independently selected from the group consisting of H, Cl, F;
**G**^{**10**} is selected from the group consisting of H, Cl, F, CH₃, -CHO;
each **G**^{**11**} is independently selected from the group consisting of H, OH, , Cl; two G¹¹ can form a carbonyl group with the C¹¹ atom;
each **G**^{**13**} is independently selected from the group consisting of H, CH₃;
each **G**^{**16**} is independently selected from the group consisting of H, CH₃, OH; two G¹⁶ can form a vinyl group with the C¹⁶ atom;
each **G**^{**17**} is independently selected from the group consisting of H, OH and a monovalent radical comprising from 1 to 20 carbon atoms and from 0 to 5 oxygen, sulfur, nitrogen, halogen atoms; preferably it is H, OH, CH₃, C≡CH, CO-R-OH, CO-RH, CO-R-Cl, OCO-RH, CO-COO-RH, R-COOH, CH(OH)R-OH, COO-R-Cl, OC(O)O-RH, CO-R-SH, CO-R-O-CO-R-N(CH₂CH₃)₂, CO-SCH₂F, CO-R-OCORH, 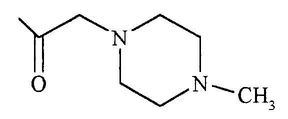
wherein
R is a C₁-C₂₀ linear or branched alkylene radical, and 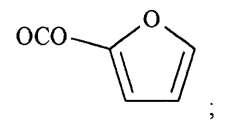
two G¹⁷ can form a carbonyl group with the C¹⁷ atom;
one G¹⁶ can unite with a G¹⁷ group to form, together with C¹⁶ and C¹⁷ the following groups: 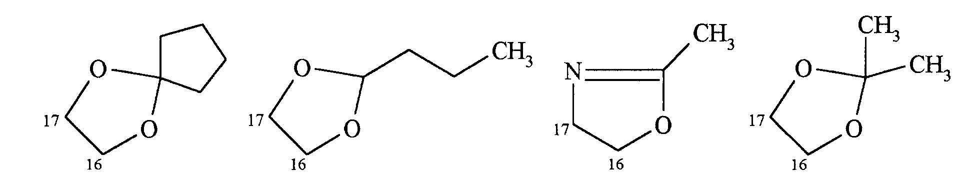 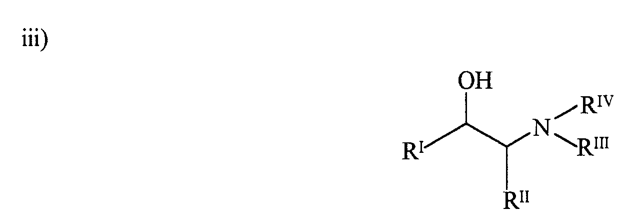
**R**^{**I**} is monovalent radical comprising from 6 to 20 carbon atoms and from 0 to 6 heteroatoms selected from oxygen, nitrogen, sulfur, chlorine, bromine, fluorine;
**R**^{**II**} is selected from the group consisting of hydrogen and linear or branched alkyl having from 1 to 4 carbon atoms;
**R**^{**III**} is selected from the group consisting of hydrogen and linear or branched alkyl having from 1 to 4 carbon atoms;
**R**^{**IV**} is selected from the group consisting of hydrogen, a linear or branched alkyl having from 1 to 4 carbon atoms and a substituted aryl; preferably R^{IV} is selected from the group consisting of tert-butyl and isopropyl; 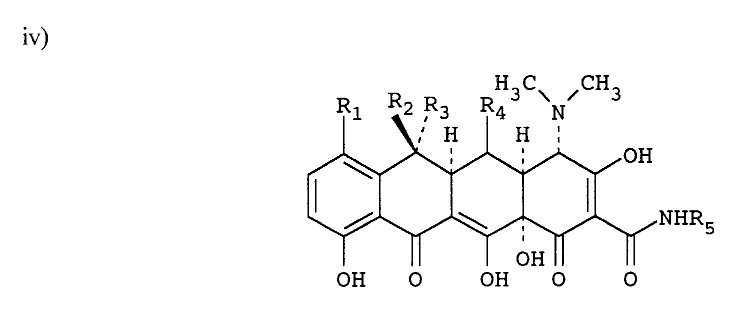
wherein:
**R**_{**1**} is selected from the group consisting of H, Cl and dimethylamino,
**R**_{**2**} is selected from the group consisting of H, OH,
**R**_{**3**} is selected from the group consisting of H, CH₃,
R₂ and R₃ together can be a methylene group (CH₂=),
**R**_{**4**} is selected from the group consisting of H, OH,
**R**_{**5**} is selected from the group consisting of H, CH₂OH and a monovalent radical containing from 5 to 20 carbon atoms and from 1 to 8 nitrogen atoms; the radical can further comprise other functional groups such as carboxyl and hydroxyl. 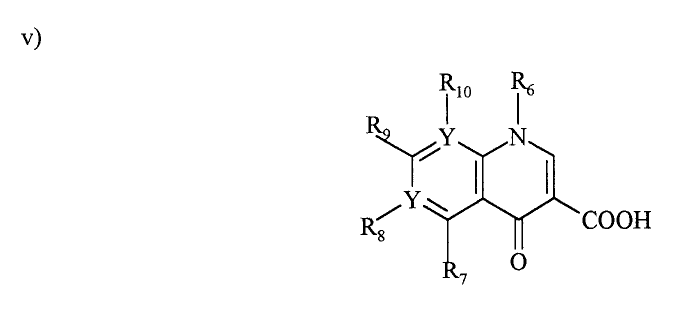
wherein
each **Y** is independently selected from the group consisting of C and N,
**R**_{**6**} is selected from the group consisting of cyclopropyl, phenyl, 4-fluorophenyl, 2,4-difluorophenyl, 2-fluoroethyl and ethyl;
**R**_{**7**} is selected from the group consisting of H, amino, methyl,
**R**_{**8**} is selected from the group consisting of H and F;
**R**_{**9**} is selected from the group consisting of H, methyl and a monovalent radical containing from 1 to 20 carbon atoms and from 1 to 4 nitrogen atoms;
**R**_{**10**} is selected from the group consisting of H, Cl and F;
R₆ e R₁₀ can unite to form an optionally substituted six membered ring optionally containing up to two heteroatoms selected from the group consisting of oxygen and sulfur: 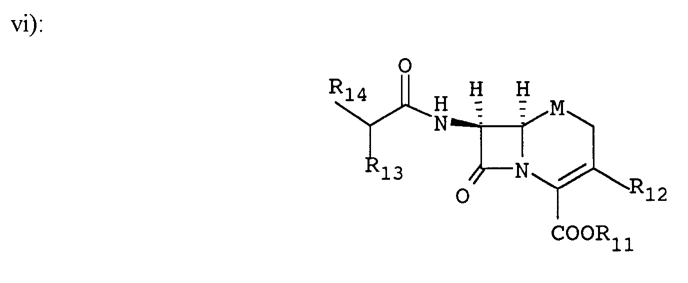
wherein
**M** is selected from the group consisting of sulfur, carbon or oxygen;
**R**_{**11**} is selected from the group consisting of H, pivaloyloxymethyl,
**R**_{**12**} is selected from the group consisting of chlorine and a monovalent radical containing from 1 to 5 carbon atoms, from 0 to 5 nitrogen atoms and from 0 to 1 sulfur atoms;
**R**_{**13**} is selected from the group consisting of amino, hydroxyl and monovalent radical containing from 1 to 10 carbon atoms, from 0 to 5 oxygen atoms and from 0 to 5 nitrogen atoms;preferably it is selected from the group consisting of amino, hydroxyl, carboxyl and
**R**_{**14**} is an unsaturated C₆ ring, optionally substituted; 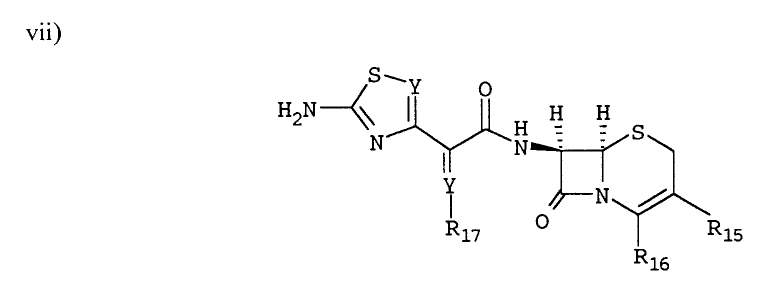
wherein:
each **Y** is independently selected from the group consisting of carbon and nitrogen
**R**_{**15**} is selected from the group consisting of hydrogen and a monovalent radical containing from 1 to 12 carbon atoms, from 0 to 4 oxygen atoms, from 0 to 5 nitrogen atoms and from 0 to 3 sulfur atoms;
**R**_{**16**} is a monovalent radical containing from 1 to 10 carbon atoms and from 2 to 8 oxygen atoms; preferably it is selected from the group consisting of carboxyl, (CH₃)₃CCOOCH₂OCO- and (CH₃)₂CHOCOOCH(CH₃)OCO-; when R₁₅ is a quaternary ammonium cation, R₁₆ is optionally a -COO⁻;
**R**_{**17**} is selected from the group consisting of -OH and a monovalent radical containing from 1 to 12 carbon atoms and from 0 to 4 oxygen atoms, preferably it is selected from the group consisting of -OH, -OCH₃, -CH₂CH₃, -OCH₂COOH, -CH₂COOH, OC(CH₂)₃-COOH. 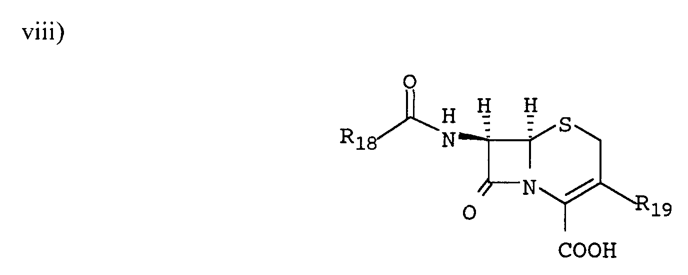
wherein:
**R**_{**18**} is a monovalent radical containing from 1 to 20 carbon atoms, from 0 to 4 oxyger atoms, from 0 to 5 nitrogen atoms, from 0 to 3 sulfur atoms and from 0 to 3 chlorine atoms;
**R**_{**19**} is selected from the group consisting of H and a monovalent radical containing from 1 to 10 carbon atoms, from 0 to 4 oxygen atoms, from 0 to 6 nitrogen atoms and from 0 to 3 sulfur atoms; 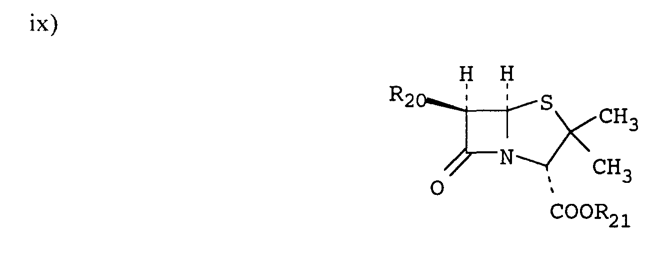
wherein:
**R**_{**20**} is a monovalent radical containing from 1 to 20 carbon atoms, from 0 to 8 oxygen atoms, from 0 to 5 nitrogen atoms, from 0 to 3 sulfur atoms, from 0 to 3 fluorine atoms and from 0 to 3 chlorine atoms;
**R**_{**21**} is selected from the group consisting of H and a monovalent radical containing from 1 to 20 carbon atoms, from 0 to 8 oxygen atoms, from 0 to 5 nitrogen atoms and from 0 to 3 sulfur atoms; 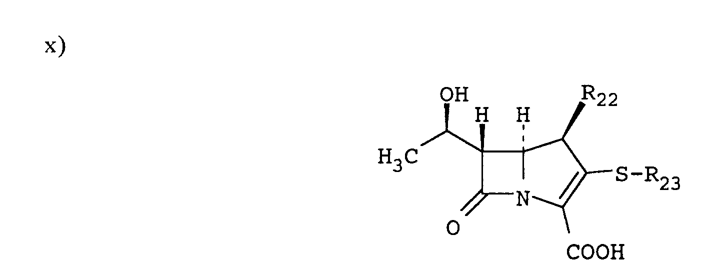
wherein:
**R**_{**22**} is selected from the group consisting of H and methyl;
**R**_{**23**} a monovalent radical containing from 1 to 10 carbon atoms, from 0 to 4 oxygen atoms and from 1 to 5 nitrogen atoms; 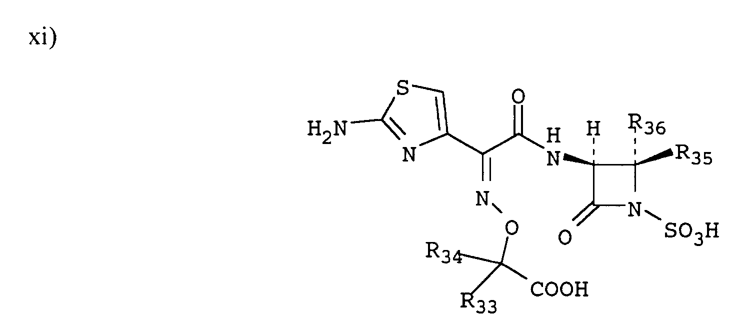
wherein:
R₃₃ , R₃₄ and R₃₆ are independently selected from the group consisting of H and CH₃;
R₃₅ is selected from the group consisting of H and -CH₂OCONH₂, 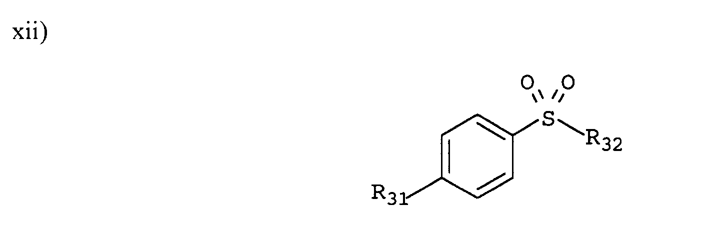
wherein:
**R**_{**31**} is selected from the group consisting of -NH₂, -CH₂NH₂ and -NHCH₂Ph
**R**_{**32**} is selected from the group consisting of -NH₂, -NHR₂₆ and a monovalent radical containing from 1 to 20 carbon atoms, from 0 to 5 oxygen atoms, from 0 to 5 nitrogen atoms and from 0 to 3 sulfur atoms; wherein **R**_{**26**} is a monovalent radical containing from 1 to 20 carbon atoms, from 0 to 8 oxygen atoms, from 0 to 5 nitrogen atoms, from 0 to 3 sulfur atoms and from 0 to 3 chlorine atoms; 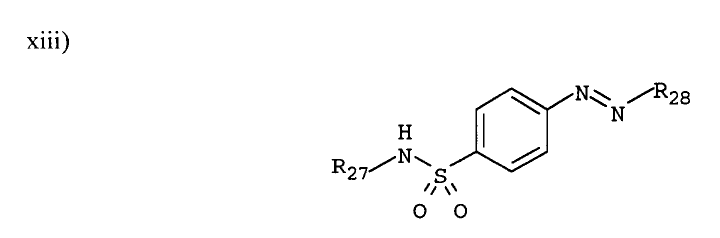
wherein:
R₂₇ is selected from the group consisting of H and 4,6-dimethyl-2-pyrimidinyl;
R₂₈ is a phenyl group substituted in at least 2 of the positions 2, 3, 4 and 6 by a group selected from hydroxyl, carboxyl and amino; 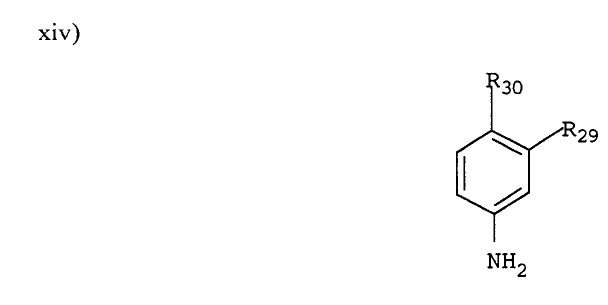
wherein:
**R**_{**29**} is selected from the group consisting of hydrogen and hydroxyl
**R**_{**30**} is selected from the group consisting of carboxyl, phenoxycarbonyl, 4-(amino)phenylsulfinyl, hydrazinocarbonyl; 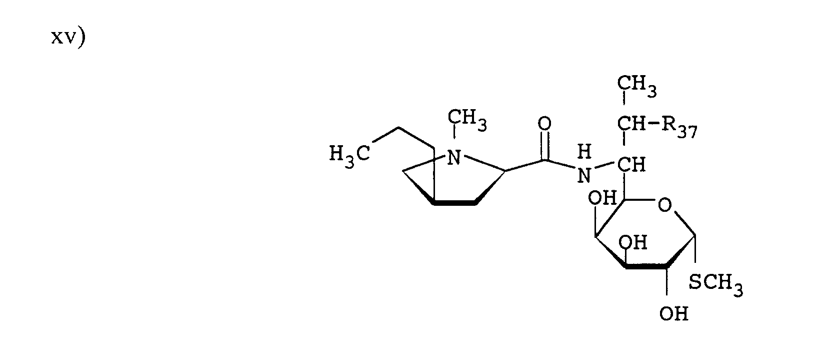
wherein:
**R**_{**37**} is selected from the group consisting of Cl and -OH; 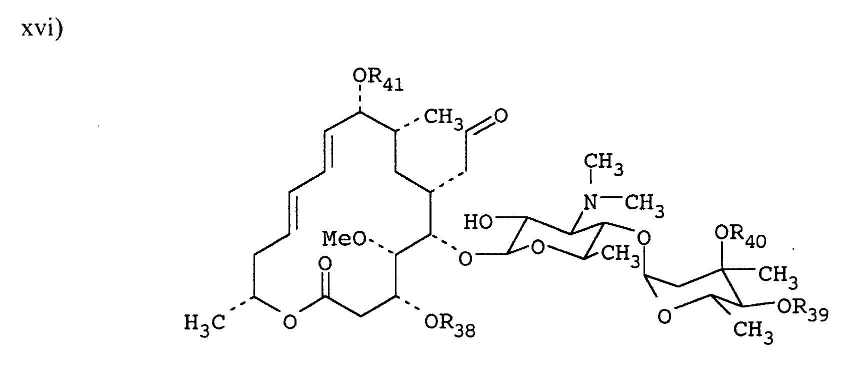
wherein:
**R**_{**38**} **R**_{**39**} **R**_{**40**} are independently selected from the group consisting of H and acyl; preferably they are selected from the group consisting of H, acetyl, propionyl, butyrryl, valeryl
**R**_{**41**} is independently selected from the group consisting of H and 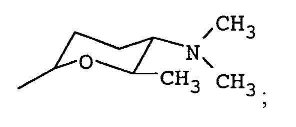 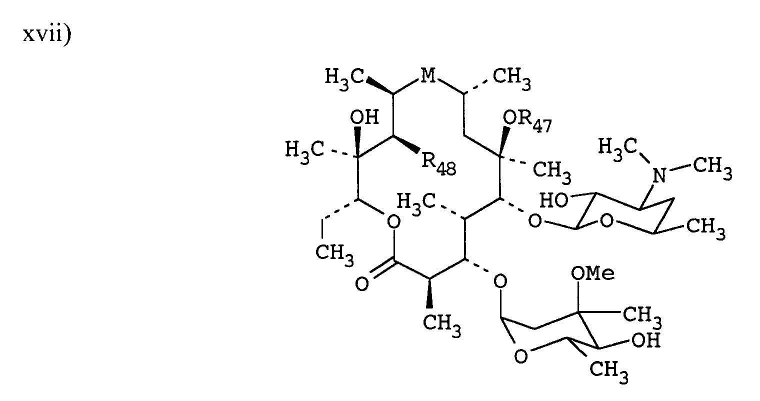
wherein:
**R**_{**47**} is selected from the group consisting of H and -CH₃
**M** is selected from the group consisting of CO, N-methyl-aminomethylene and -CH(NHR₄₉)- wherein **R**_{**49**} is a substituted methylene bridge connecting N with **R**_{**48**} **R**_{**48**} is hydroxyl or, when **M** is -CH(NHR₄₉)-, is -O-;
Preferably **R**_{**49**} is 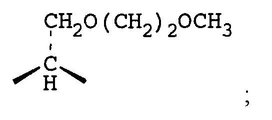 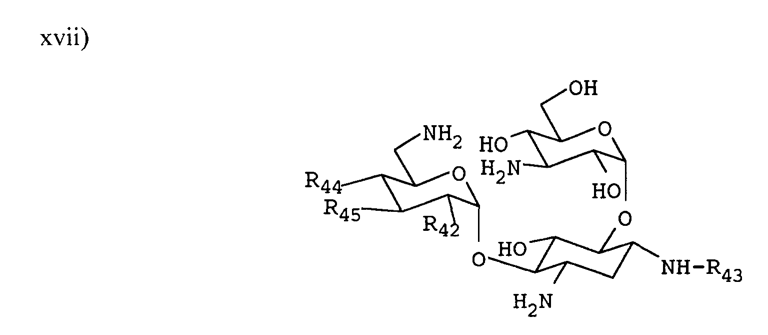 wherein:
**R**_{**42**} is selected from the group consisting of hydroxyl and amino;
**R**_{**43**} is selected from the group consisting of hydrogen, (R) and (S)-4-amino-2-hydroxybutyrryl
**R**_{**44**} and **R**_{**45**} are independently selected from the group consisting of hydrogen and hydroxyl. 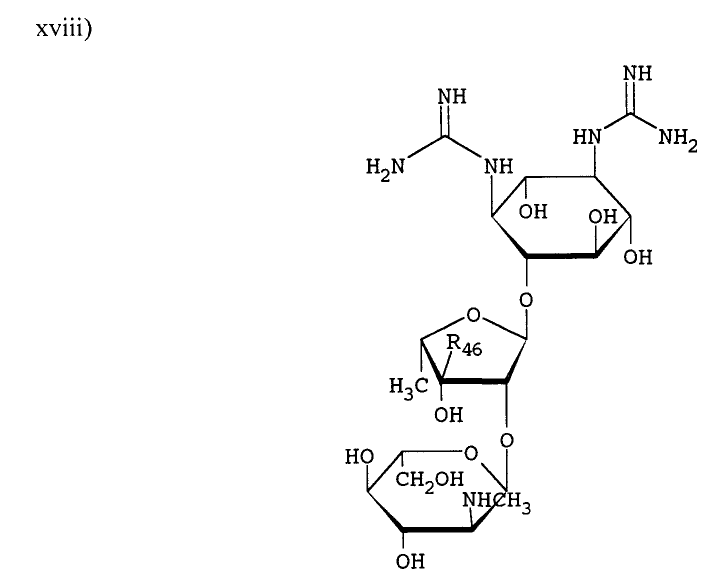
wherein:
**R**_{**46**} is selected from the group consisting of -CH₂OH and -CHO; 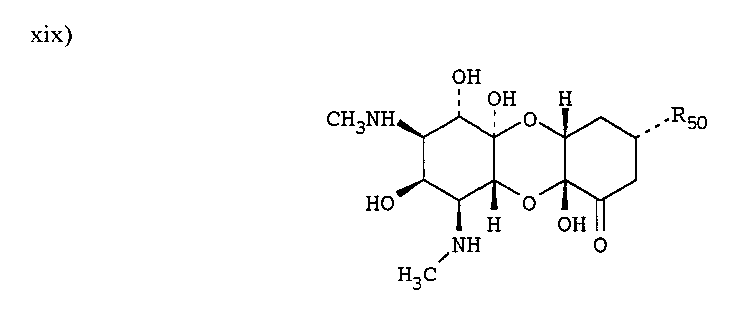
wherein:
**R**_{**50**} is a C₁-C₄ alkyl, preferably it is selected from the group consisting of methyl and n-butyl. 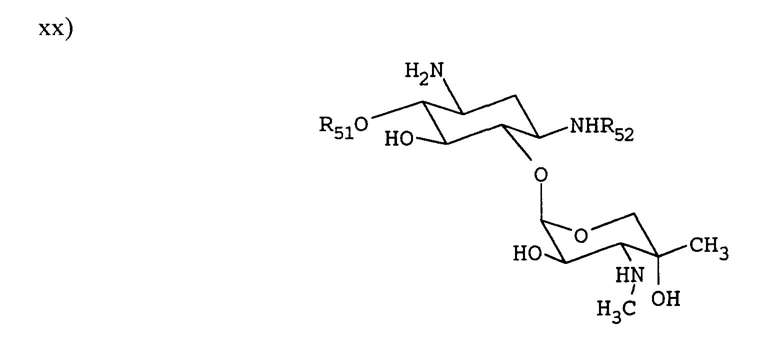
wherein:
**R**_{**51**} is independently selected from the group consisting of 3-amino-6-(aminomethyl)-3,4-dihydro-2H-pyran-2-yl and 2-amino-2,3,4,6-tetradeoxy-6-(methylamino)-α-D-eritro-hexopyranosyl,
**R**_{**52**} is selected from the group consisting of H and -CH₂CH₃. 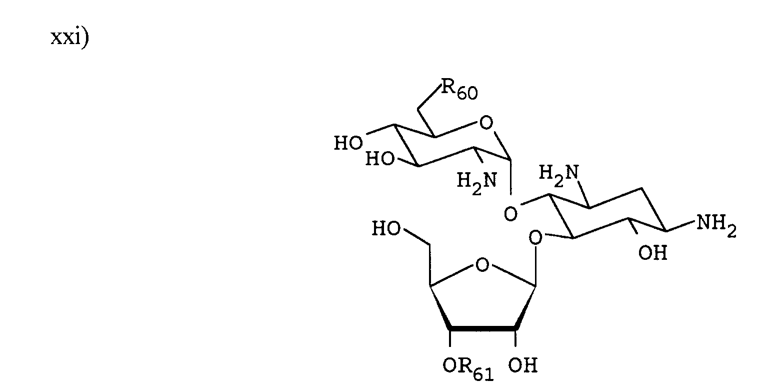
wherein:
**R**_{**60**} is selected from the group consisting of -OH and -NH₂;
**R**_{**61**} is selected from the group consisting of H, 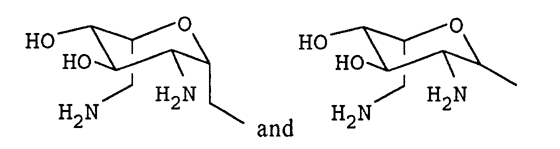 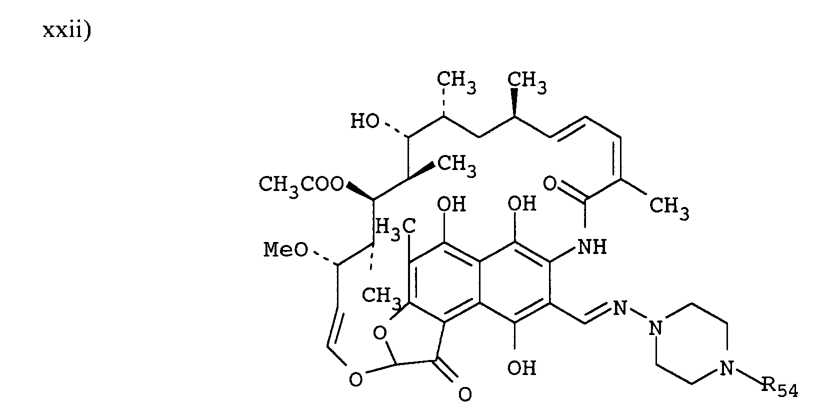
wherein **R**_{**54**} is a C₁-C₄ linear or cyclic alkyl, preferably it is selected from the group consisting of methyl and cyclopropyl.

10. Composition according to claim 8 wherein the drug is selected from the group consisting of:
Aspirin, Salicylic acid, Mesalamine, Acetylsalicylsalicylic acid, Paracetamol, Etodolac, Pirazolac, Tolmetin, Bromefenac, Fenbufen, Mofezolac, Diclofenac, Pemedolac, Sulindac, Ketorolac, Indomethacin, Suprofen, Ketoprofen, Tiaprofenic acid, Fenoprofen, Indoprofen, Carprofen, Naproxen, Loxoprofen, Ibuprofen, Pranoprofen, Bermoprofen, CS-670, Zaltoprofen, Tenoxicam, Piroxicam, Meloxicam, Tenidap, Aceclofenac, Acemetacin, 5-amino-acetylsalicylic acid, Alclofenac, Alminoprofen, Amfenac, Bendazac, α-bisabolol, Bromosaligenin, Bucloxic acid, Butibufen, Cinmetacin, Clidanac, Clopirac, Diflunisal, Ditazol, Enfenamic acid, Etofenamate, Felbinac, Fenclozic acid, Fendosal, Fentiazac, Fepradinol, Flufenamic acid, Flunixin, Flunoxaprofen, Flurbiprofen, Glucametacin, Glycol salicilate, Ibuproxam, Isofezolac, Isoxepac, Isoxicam, Lornoxicam, Meclofenamic acid, Mefenamic acid, Metiazinic acid, Niflunic acid, Oxaceprol, Oxaprozin, Oxyphenbutazone, Parsalmide, Perisoxal, Olsalazine, Pirprofen, Protizinic acid, Salacetamide, Salicilamide O-acetic acid, Salsalate, Suxibuzone, Tiaramide, Tinoridine, Tolfenamic acid, Tropesin, Xenbucin, Ximoprofen, Zomepirac, Tomoxiprol.
